# EUROPEAN PATENT SPECIFICATION

(11) **EP 1 833 811 B1**
(45) Date of publication and mention of the grant of the patent: **02.06.2010**
(21) Application number: 05820756.4
(22) Date of filing: 21.12.2005
(51) Int. Cl.: C07D 307/84, A61K 31/343, A61P 25/00

(54) **OXYGEN CONTAINING HETEROCYCLES AS GLYCINE TRANSPORTER INHIBITING COMPOUNDS**
SAUERSTOFFHALTIGE HETEROCYCLEN ALS GLYCINTRANSPORTERINHIBIERENDE VERBINDUNGEN
HÉTÉROCYCLES OXYGÉNÉS EN TANT QUE COMPOSÉS INHIBITEURS DE TRANSPORTEURS DE GLYCINE

(30) Priority: 23.12.2004 GB 0428232
(43) Date of publication of application: 19.09.2007
(73) Proprietor: GLAXO GROUP LIMITED, Greenford, Middlesex UB6 ONN (GB)
(72) Inventor: BOZZOLI, Andrea, I-37135 Verona (IT); BRANCH, Clive Leslie, GlaxoSmithKline, Harlow Essex CM19 5AW (GB); MACRITCHIE, Jacqueline Anne, Saffron Walden Essex CB10 1XL (GB); MARSHALL, Howard Robert, GlaxoSmithKline, Harlow Essex CM19 5AW (GB); PORTER, Roderick Alan, GlaxoSmithKline, Harlow Essex CM19 5AW (GB); SPADA, Simone, I-37135 Verona (IT)
(74) Representative: Kondo, Rie
(86) International application number: PCT/GB2005/004969
(87) International publication number: WO 2006/067437

(56) References cited:
- WO-A-03/055478
- US-A- 2 746 959

## Description

The present invention relates to glycine transporter inhibiting compounds, their use in the manufacture of medicaments for treating neurological and neuropsychiatric disorders, in particular psychoses, dementia or attention deficit disorder. The invention further comprises processes to make these compounds and pharmaceutical formulations thereof.

Molecular cloning has revealed the existence in mammalian brains of two classes of glycine transporters, termed GlyT1 and GlyT2. GlyT1 is found predominantly in the forebrain and its distribution corresponds to that of glutamatergic pathways and NMDA receptors (Smith, et al., Neuron, 8, 1992: 927-935). Molecular cloning has further revealed the existence of three variants of GlyT1, termed GlyT-la, GlyT-1b and GlyT-1c (Kim et al., Molecular Pharmacology, 45, 1994: 608-617), each of which displays a unique distribution in the brain and peripheral tissues. The variants arise by differential splicing and exon usage, and differ in their N-terminal regions. GlyT2, in contrast, is found predominantly in the brain stem and spinal cord, and its distribution corresponds closely to that of strychnine-sensitive glycine receptors (Liu et al., J. Biological Chemistry, 268, 1993: 22802-22808; Jursky and Nelson, J. Neurochemistry, 64, 1995 : 1026-1033). Another distinguishing feature of glycine transport mediated by GlyT2 is that it is not inhibited by sarcosine as is the case for glycine transport mediated by GlyT1. These data are consistent with the view that, by regulating the synaptic levels of glycine, GlyT1 and GlyT2 selectively influence the activity of NMDA receptors and strychnine-sensitive glycine receptors, respectively.

NMDA receptors are critically involved in memory and learning (Rison and Staunton, Neurosci. Biobehav. Rev., 19 533-552 (1995); Danysz et al, Behavioral Pharmacol., 6 455-474 (1995)); and, furthermore, decreased function of NMDA-mediated neurotransmission appears to underlie, or contribute to, the symptoms of schizophrenia (Olney and Farber, Archives General Psychiatry, 52, 998-1007 (1996). Thus, agents that inhibit GlyT1 and thereby increase glycine activation of NMDA receptors can be used as novel antipsychotics and anti-dementia agents, and to treat other diseases in which cognitive processes are impaired, such as attention deficit disorders and organic brain syndromes. Conversely, over-activation of NMDA receptors has been implicated in a number of disease states, in particular the neuronal death associated with stroke and possibly neurodegenerative diseases, such as Alzheimer's disease, multi-infarct dementia, AIDS dementia, Huntington's disease, Parkinson's disease, amyotrophic lateral sclerosis or other conditions in which neuronal cell death occurs, such as stroke or head trauma. Coyle & Puttfarcken, Science, 262, 689-695 (1993); Lipton and Rosenberg, New Engl. J. of Medicine, 330, 613-622 (1993); Choi, Neuron, 1, 623-634 (1988). Thus, pharmacological agents that increase the activity of GlyT1 will result in decreased glycine-activation of NMDA receptors, which activity can be used to treat these and related disease states. Similarly, drugs that directly block the glycine site of the NMDA receptors can be used to treat these and related disease states.
Glycine transport inhibitors are already known in the art, for example as disclosed in published international patent application WO03/055478 (SmithKline Beecham). US-A-2746 959 infact discloses phenylmethyl carboxamide similar to the present compounds but does not-concern glycine transport inhibitors.
However, there still remains the need to identify further compounds that can inhibit GlyT1 transporters, including those that inhibit GlyT1 transporters selectively over GlyT2 transporters.

It is now found that a novel class of compounds inhibit GlyT1 transporters and are thus useful in the treatment of certain neurological and neuropsychiatric disorders, including schizophrenia.

Thus, in a first aspect, there is provided a compound of formula (I) or a salt or solvate thereof: 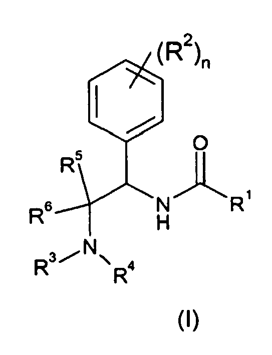 wherein:
R¹ is a group selected from: 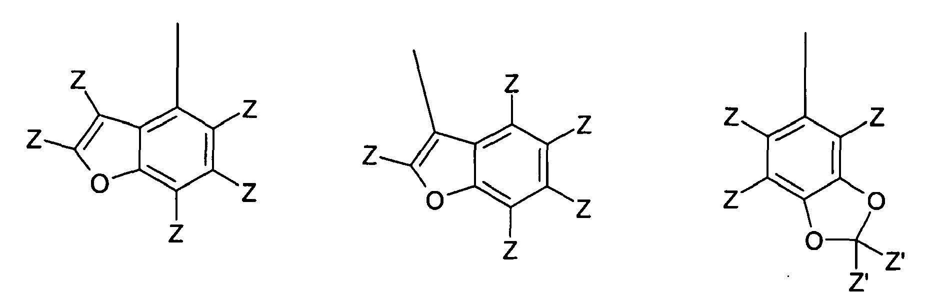 and wherein
Each Z is independently selected from hydrogen, halogen, C₃₋₇cycloalkyl, C₁₋₄alkyl, haloC₁₋₄alkyl, C₁₋₄alkoxyC₁₋₄alkyl, C₁₋₄alkoxy, haloC₁₋₄alkoxy, C₁₋₄alkylthio, haloC₁-₄alkylthio, C₃₋₇cycloalkylC₁₋₄alkoxy, C₁₋₄alkylsulphoxy, C₁₋₄ alkylsulphonyl, and cyano;
and each Z' is independently selected from hydrogen, fluoro or C₁₋₄alkyl;
R² is selected from the group consisting of halogen, cyano, C₁₋₄alkoxy, C₁₋₄alkyl, haloC₁₋₄alkyl, haloC₁₋₄alkoxy, C₃₋₇cycloalkyl, C(O)NR⁹R¹⁰, (where each of R⁹ and R¹⁰ is independently hydrogen or C₁₋₄alkyl, or R⁹ and R¹⁰ together with the nitrogen atom to which they are attached form a 4-, 5-, 6- or 7-membered saturated carbocyclic ring, the 4-, 5-, 6- or 7-membered saturated ring optionally further comprising an additional heteroatom group selected from O, N and S(O)ₘ (where m is 0, 1, or 2)), C₃₋₇cycloalkylC₁₋₄alkoxy, C₁₋₄alkylthio, and haloC₁₋₄alkylthio;
n is 0, 1 or 2.
R³ and R⁴ are independently selected from hydrogen, C₁₋₄alkyl, optionally substituted with one or more groups Y; or R³ and R⁴ together with the nitrogen atom to which they are attached form a saturated or partially unsaturated carbocyclic 4-, 5- 6-or 7-membered ring optionally substituted with a group Y';
Y is selected from the group consisting of C₁₋₄alkoxy, hydroxy, haloC₁₋₄alkoxy and C₃₋₅cycloalkyl and C₅₋₁₀ aryl;
Y' is selected from the group consisting of C₁₋₄alkyl, C₁₋₄alkoxy, halogen, hydroxy, haloC₁₋₄alkoxy, C₃₋₅cydoalkyl and C₅₋₁₀aryl or Y' forms a -CH₂- or -CH₂-CH₂- bridge between two atoms on the 4-, 5- or 6- membered ring;
R⁵ and R⁶ are independently C₁₋₄alkyl, optionally substituted with one or more groups X; or
R⁵ and R⁶ together with the carbon atom to which they are attached form a saturated 5- or
6-membered carbocyclic ring optionally substituted with one or more groups X', in the case of R⁵ and R⁶ together with the carbon atom to which they are attached forming a 5-membered saturated carbocyclic ring, that ring may optionally further comprise an additional heteroatom group selected from O N and S(O)ₘ; where m = 0, 1 or 2.

X is selected from the group consisting of halogen, hydroxy, C₁₋₄alkoxy, haloC₁₋₄alkyl, haloC₁₋₄alkoxy and C₅₋₁₀aryl; and

X' is selected from the group consisting of halogen, hydroxy, C₁₋₄alkoxy, haloC₁₋₄alkyl, haloC₁₋₄alkoxy and C₅₋₁₀aryl.

As used herein, the term "alkyl" refers to a straight or branched alkyl group in all isomeric forms. Examples of C₁₋₄alkyl include methyl, ethyl, propyl, isopropyl, butyl, isobutyl, sec-butyl and tert-butyl.

As used herein, the term "cycloalkyl" refers to a non-aromatic cyclic saturated hydrocarbon ring. Examples of C₃₋₇cycloalkyl include cyclopropyl, cyclobutyl, cyclopentyl, cyclohexyl and cycloheptyl.

As used herein, the term "alkoxy" refers to the group -O-alkyl wherein alkyl is as defined above.

As used herein, the term "alkylthio" refers to the group -S-alkyl wherein alkyl is as defined above.

As used herein, the term "C₅₋₁₀aryl" refers to a 5- or 6- membered monocyclic aromatic group or a 8- to 10- membered bicyclic aromatic group. Examples of C₅₋₁₀aryl include phenyl, indenyl, azulenyl and naphthyl.

As used herein, the terms "halogen" and its abbreviation "hal" refer to fluorine, chlorine, bromine, or iodine.

As used herein, the term "haloalkyl" refers to an alkyl group as defined above which is substituted with any number of fluorine, chlorine, bromine, or iodine atoms, including with mixtures of those atoms. A haloalkyl group may, for example contain 1, 2 or 3 halogen atoms. For example, a haloalkyl group may have all hydrogen atoms replaced with halogen atoms. Examples of haloalkyl groups include fluoromethyl, difluoromethyl and trifluoromethyl.

As used herein, the term "salt" refers to any salt of a compound according to the present invention prepared from an inorganic or organic acid or base, quaternary ammonium salts and internally formed salts. Physiologically acceptable salts are particularly suitable for medical applications because of their greater aqueous solubility relative to the parent compounds. Such salts must clearly have a physiologically acceptable anion or cation. Suitably physiologically acceptable salts of the compounds of the present invention include acid addition salts formed with inorganic acids such as hydrochloric, hydrobromic, hydroiodic, phosphoric, metaphosphoric, nitric and sulfuric acids, and with organic acids, such as tartaric, acetic, trifluoroacetic, citric, malic, lactic, fumaric, benzoic, formic, propionic, glycolic, gluconic, maleic, succinic, camphorsulfuric, isothionic, mucic, gentisic, isonicotinic, saccharic, glucuronic, furoic, glutamic, ascorbic, anthranilic, salicylic, phenylacetic, mandelic, embonic (pamoic), methanesulfonic, ethanesulfonic, pantothenic, stearic, sulfinilic, alginic, galacturonic and arylsulfonic, for example benzenesulfonic and p-toluenesulfonic, acids; base addition salts formed with alkali metals and alkaline earth metals and organic bases such as N,N-dibenzylethylenediamine, chloroprocaine, choline, diethanolamine, ethylenediamine, meglumaine (N-methylglucamine), lysine and procaine; and internally formed salts. Salts having a non-physiologically acceptable anion or cation are within the scope of the invention as useful intermediates for the preparation of physiologically acceptable salts and/or for use in non-therapeutic, for example, *in vitro,* situations. The salts may have any suitable stoichiometry. For example, a salt may have 1:1 or 2:1 stoichiometry. Non-integral stoichiometry ratios are also possible.

As used herein, the term "solvate" refers to a complex of variable stoichiometry formed by a solute (in this invention, a compound of formula (I) or a salt thereof) and a solvent. Such solvents for the purpose of the invention may not interfere with the biological activity of the solute. Examples of suitable solvents include, but are not limited to, water, methanol, ethanol and acetic acid. Preferably the solvent used is a pharmaceutically acceptable solvent. Examples of suitable pharmaceutically acceptable solvents include water, ethanol and acetic acid. Most preferably the solvent used is water.

In one embodiment, R¹ is selected from the group consisting of benzofuranyl and benzodioxolyl..

In one embodiment, Z is selected from the group consisting of hydrogen, halogen, cyano, C₁₋₄alkyl, C₁₋₄alkoxy, haloC₁₋₄alkyl, haloC₁₋₄alkoxy, C₁₋₄alkoxyC₁₋₄alkyl, C₃₋₆cycloalkyl and C₁₋₄alkylthio.

In a further embodiment, Z is selected from the group consisting of hydrogen, halogen, cyano, C₁₋₄alkyl, C₁₋₄alkoxy, haloC₁₋₄alkyl, C₁₋₄alkylthio and C₃₋₆cycloalkyl. In a further embodiment, Z is selected from the group consisting of hydrogen, C₁₋₄alkyl, C₁₋₄alkoxy, C₁₋₄alkylthio and haloC₁₋₄alkoxy. In a further embodiment, Z is selected from hydrogen, and methoxy.

In one embodiment all, or all but one, two or three groups Z are hydrogen. In one embodiment all but one or two groups Z are hydrogen. For example one group Z is not hydrogen.

In one embodiment, Z' is methyl.

In one embodiment, R² is selected from the group consisting of halogen, C₁₋₄alkoxy, C₁₋₄alkyl, haloC₁₋₄alkyl, and haloC₁₋₄alkoxy. In one embodiment, R² is selected from the group consisting of methoxy, fluoro, chloro, methyl and trifluoromethyl.

For example, n may be 0, 1 or 2. For example, n may be 0.

In one embodiment, R³ and R⁴ are both simultaneously the same C₁₋₄alkyl, the same C₁₋₄alkyl substituted with one or more groups Y, or R³ and R⁴ together with the nitrogen atom to which they are attached form a saturated 5- or 6-membered carbocyclic ring optionally substituted with a group Y'.

In one embodiment, R³ and R⁴ are both C₁₋₄alkyl, for example methyl or ethyl, for example methyl.

Y may, for example, be selected from the group consisting of C₁₋₄alkoxy, haloC₁₋₄alkoxy and C₅₋₁₀aryl. In one embodiment, Y is selected from the group consisting of C₁₋₄alkoxy and C₅₋₁₀aryl.

Y' may, for example, be selected from the group consisting of halogen, C₁₋₄alkyl, C₁₋₄alkoxy, haloC₁₋₄alkoxy and C₅₋₁₀aryl. In one embodiment, Y' is selected from the group consisting of C₁₋₄alkyl, C₁₋₄alkoxy and C₅₋₁₀aryl.

In one embodiment, R³ and R⁴ are independently selected from hydrogen, C₁₋₄alkyl (for example methyl and ethyl), optionally substituted with a group Y, or R³ and R⁴ together with the nitrogen atom to which they are attached form a saturated or partially unsaturated (for example saturated) 4-, 5-, 6- or 7-membered carbocyclic ring optionally substituted with a group Y'.

In a further embodiment, R³ and R⁴ are selected from methyl and ethyl, optionally substituted with a group Y, or R³ and R⁴ together with the nitrogen atom to which they are attached form a saturated 4-, 5- or 6-membered carbocyclic ring optionally substituted with a group Y'. For example, R³ and R⁴ are both unsubstituted methyl, or R³ and R⁴ together with the nitrogen atom to which they are attached form a saturated 5- or 6-membered carbocyclic ring, for example R³ and R⁴ are both unsubstituted methyl.

Y may, for example, be selected from the group consisting of C₁₋₄alkoxy, hydroxyl, C₃₋₅cycloalkyl and C₅₋₁₀aryl.

Y' may, for example, be selected from the group consisting of halogen and C₁₋₄alkyl or Y' may form a -CH₂- bridge between two atoms on the 5- or 6- membered ring. In a further embodiment, Y' is selected from the group consisting of halogen and C₁₋₄alkyl.

In one embodiment, R⁵ and R⁶ are both simultaneously the same C₁₋₄alkyl, the same C₁₋₄alkyl substituted with one or more groups X, or R⁵ and R⁶ together with the carbon atom to which they are attached form a saturated 5- or 6-membered carbocyclic ring optionally substituted with a group X', the 5- or 6-membered saturated carbocyclic ring optionally further comprising an additional heteroatom group selected from O, N and S(O)ₘ (where m is 0, 1, or 2);

In a further embodiment, R⁵ and R⁶ are both methyl or R⁵ and R⁶ together with the carbon atom to which they are attached form a saturated 5- or 6-membered carbocyclic ring, for example a 5-membered carbocyclic ring.

X is, for example, selected from the group consisting of halogen, C₁₋₄alkoxy, haloC₁₋₄alkyl, haloC₁₋₄alkoxy and C₅₋₁₀aryl.

In one embodiment, R⁵ and R⁶ are independently selected from methyl and ethyl, optionally substituted with one or more groups X; or R⁵ and R⁶ together with the carbon atom to which they are attached form a saturated 5- or 6-membered carbocyclic ring and in the case of R⁵ and R⁶ together with the carbon atom to which they are attached forming a carbocyclic 5-membered saturated ring, that ring may optionally further comprise an oxygen heteroatom.

For example, in one embodiment R⁵ and R⁶ are independently selected from methyl and ethyl, or R⁵ and R⁶ together with the carbon atom to which they are attached form a saturated 5- membered carbocyclic ring. For example, in a further embodiment, R⁵ and R⁶ are both methyl, or R⁵ and R⁶ together with the carbon atom to which they are attached form a saturated 5- membered carbocyclic ring. For example, in a further embodiment, R⁵ and R⁶ together with the carbon atom to which they are attached form a saturated 5-membered carbocyclic ring.

X may, for example, be selected from the group consisting of hydroxy and C₁₋₄alkoxy.

X' may, for example, be selected from the group consisting of hydroxy and C₁₋₄alkoxy.

In one embodiment, at least one of the pairs of groups R³ / R⁴ and R⁵ / R⁶ forms a cyclic group with the Nitrogen or Carbon atom to which they are respectively attached. For example, that cyclic group may be a 5-membered ring.

Accordingly, in one embodiment, the present invention provides a compound of formula (la) or a salt or solvate thereof: 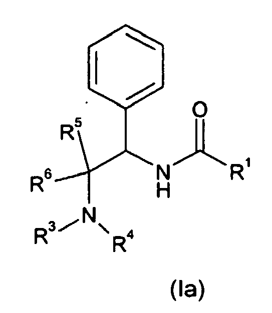 wherein:
R¹ is a group selected from: 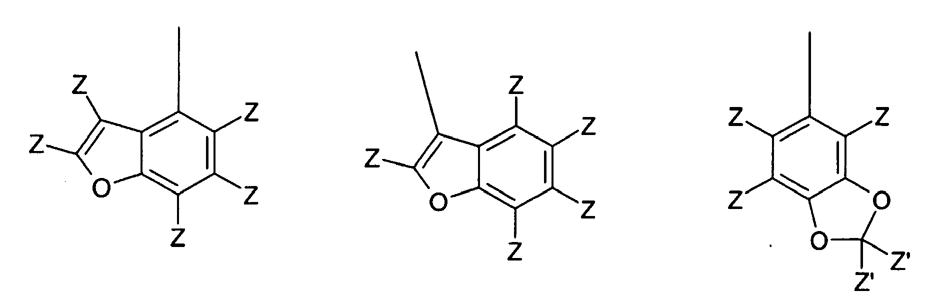
wherein
each Z is independently selected from the group consisting of hydrogen, halogen, cyano, C₁₋₄alkyl, C₁₋₄alkoxy, haloC₁₋₄alkyl, haloC₁₋₄alkoxy, C₁₋₄alkoxyC₁₋₄alkyl, C₁₋₄alkylthio, C₃₋₆cycloalkyl, ;
each Z' is methyl;
R³ and R⁴ are independently selected from hydrogen, C₁₋₄alkyl (for example methyl and ethyl), optionally substituted with a group Y, or R³ and R⁴ together with the nitrogen atom to which they are attached form a saturated or partially unsaturated (for example saturated) 4-, 5-, 6- or 7-membered carbocyclic ring optionally substituted with a group Y';
Y is selected from the group consisting of C₁₋₄alkoxy, hydroxyl, C₃₋₅cycloalkyl and C₅₋₁₀aryl;
Y' is selected from the group consisting of halogen and C₁₋₄alkyl;
R⁵ and R⁶ are independently selected from methyl and ethyl, optionally substituted with one or more groups X; or R⁵ and R⁶ together with the carbon atom to which they are attached form a saturated 5- or 6-membered carbocyclic ring and in the case of R⁵ and R⁶ together with the carbon atom to which they are attached forming a carbocyclic 5-membered saturated ring, that ring may optionally further comprise an oxygen heteroatom; and

X is selected from the group consisting of hydroxy and C₁₋₄alkoxy.

All embodiments and features of compounds of formula (I) apply to compounds of formula (la).

Examples of compounds of the invention include Examples 1 to 5 shown below, as well as salts and solvates thereof.

The compounds of formula (I) may have the ability to crystallise in more than one form. This is a characteristic known as polymorphism, and it is understood that such polymorphic forms ("polymorphs") are within the scope of formula (I). Polymorphism generally can occur as a response to changes in temperature or pressure or both and can also result from variations in the crystallisation process. Polymorphs can be distinguished by various physical characteristics known in the art such as x-ray diffraction patterns, solubility, and melting point.

Certain of the compounds described herein may exist in stereoisomeric forms (i.e. they may contain one or more asymmetric carbon atoms or may exhibit *cis-trans* isomerism).
The individual stereoisomers (enantiomers and diastereoisomers) and mixtures of these are included within the scope of the present invention. Likewise, it is understood that compounds of formula (I) may exist in tautomeric forms other than that shown in the formula and these are also included within the scope of the present invention.

As referred to above, individual enantiomers of compounds of formula (I) may be prepared. In a preferred embodiment, an optically pure enantiomer is desired. The term "optically pure enantiomer" means that the compound contains greater than about 90 % of the desired isomer by weight, preferably greater than about 95 % of the desired isomer by weight, and most preferably greater than about 99 % of the desired isomer by weight, said weight percent based upon the total weight of the isomer(s) of the compound. In some cases, one enantiomer of a particular structure may have a significantly higher activity than the other enantiomer of the same structure. Chirally pure, or chirally enriched compounds may be prepared by chirally selective synthesis or by separation of enantiomers. The separation of enantiomers may be carried out on the final product or, alternatively on a suitable intermediate.

The compounds of this invention may be made by a variety of methods, including standard chemistry. Any previously defined variable will continue to have the previously defined meaning unless otherwise indicated. Illustrative general synthetic methods are set out below and then specific compounds of the invention are prepared in the working Examples.

Compounds of general formula (I) may be prepared by methods known in the art of organic synthesis as set forth in part by the following synthesis schemes. It is also recognised that in all of the schemes described below, it is well understood that protecting groups for sensitive or reactive groups are employed where necessary in accordance with general principles of chemistry. Protecting groups are manipulated according to standard methods of organic synthesis (T. W. Greene and P. G. M. Wuts (1991) Protecting Groups in Organic Synthesis, John Wiley & Sons). These groups are removed at a convenient stage of the compound synthesis using methods that are readily apparent to those skilled in the art. The selection of processes as well as the reaction conditions and order of their execution shall be consistent with the preparation of compounds of formula (I). Those skilled in the art will recognise if a stereocentre exists in compounds of formula (I). Accordingly, the present invention includes both possible stereoisomers and includes not only racemic compounds but the individual enantiomers as well. Where the stereochemistry is indicated as being variable at certain positions, a mixture of stereoisomers may be obtained, this mixture having been separated where indicated. Stereoisomers may be separated by high-performance liquid chromatography or other appropriate means. When a compound is desired as a single enantiomer, it may be obtained by stereospecific synthesis or by resolution of the final product or any convenient intermediate. Resolution of the final product, an intermediate, or a starting material may be effected by any suitable method known in the art. See, for example, Stereochemistry of Organic Compounds by E. L. Eliel, S. H. Wilen, and L. N. Mander (Wiley-Interscience, 1994).

Typical reaction routes for the preparation of a compound of formula (I) as hereinbefore defined, are shown in the following schemes. The starting materials and reagents are known to the skilled person in the art and/or can be prepared using methods known in the art.

Compounds of formula (I) can be synthesised by known methods; for example by, but not limited to, the synthetic route outlined in the scheme below 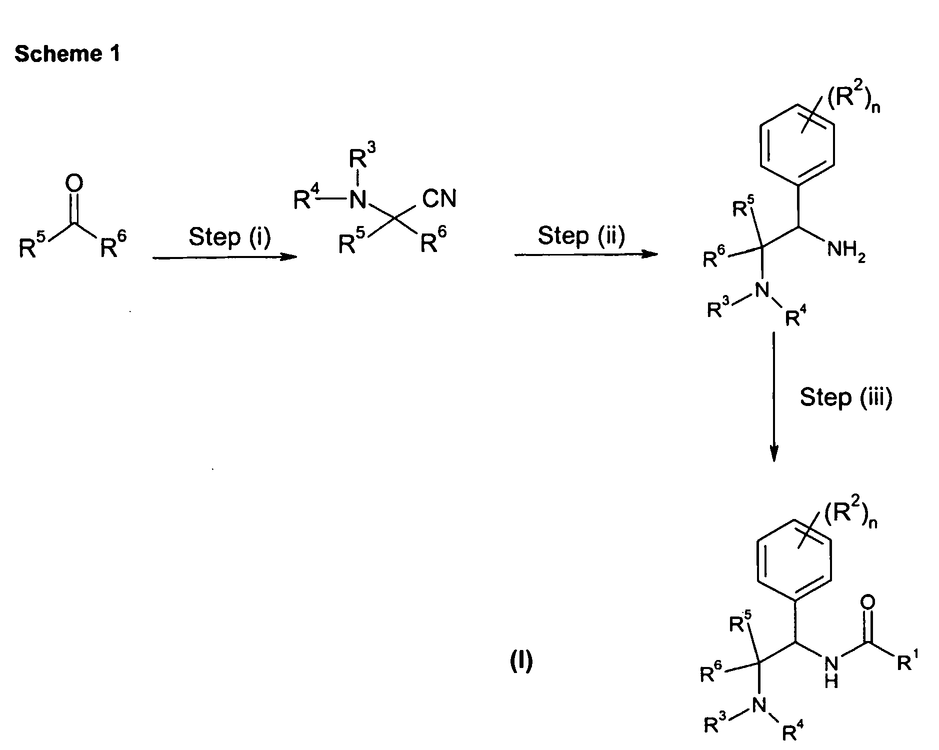 wherein n, R¹, R², R³, R⁴, R⁵ and R⁶ are as defined for the compound of formula (I).

Step (i) is carried out for example by reaction of a ketone with an amine or amine salt in the presence of inorganic cyanide, for example potassium cyanide, in solvent such as water or by reaction of a ketone with an amine and trimethylsilyl cyanide in either the absence of solvent or in a solvent such as acetic acid.

Step (ii) can be achieved by successive reaction with an appropriate organometallic reagent, for example phenyllithium, in a suitable inert solvent for example tetrahydrofuran, followed by reduction with a reducing agent, for example, sodium borohydride in a suitable solvent, for example methanol.

Acylation step (iii) can be achieved by reaction with a compound of formula (III): 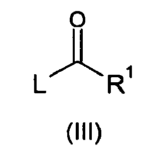 wherein R¹, is as defined in formula (I) and L represents a suitable leaving group. Examples of leaving groups include halogen, hydroxy, OC(=O)alkyl, OC(=O)O-alkyl and OSO₂Me. L may be halogen and acylation in step (iii) may be carried out in an inert solvent such as dichloromethane, in the presence of a base such as triethylamine. When L represents hydroxy, the reaction preferably takes place in an inert solvent such as dichloromethane in the presence of a coupling reagent, for example a diimide reagent such as N,N dicyclohexylcarbodiimide (DCC), N-(3-(dimethylamino)propyl)-N-ethylcarbodiimide hydrochloride (EDC), polymer-supported EDC, polymer-supported DCC or O-(7-azabenzotriazol-1-yl)-1,1,3,3-tetramethyluronium hexafluoro phosphate (HATU).

Within the scheme there is scope to convert a group R³ into another group R³ and similarly for groups R¹, R², R⁴, R⁵ and R⁶.

Accordingly, in a second aspect, the present invention provides a method of preparing a compound of formula (I), comprising the step of reacting a compound of formula (II): 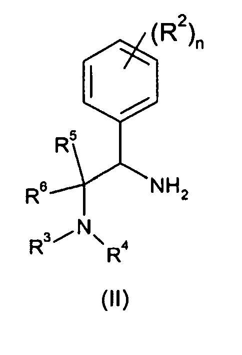 wherein R², R³, R⁴, R⁵ and R⁶ are as defined in formula (I), with a compound of formula (III): 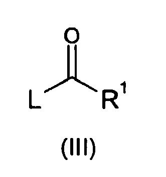 wherein R¹ is as defined in formula (I) and L represents a suitable leaving group;
and thereafter optionally:
- removing any protecting groups and/or
- converting a compound of formula (I) into another compound of formula (I) and/or
- forming a salt or solvate.

Suitable leaving groups L include halogen, hydroxy, OC(=O)alkyl, OC(=O)O-alkyl and OSO₂Me.

Compounds of formula (I) can be converted into further compounds of formula (I) using standard techniques. For example, and by way of illustration rather than limitation, possible conversion reactions include acylation with an appropriate acylating agent such as acetyl chloride, alkylation using an appropriate alkylating reagent such as methyl iodide, and sulfonylation using a sulfonylating agent such as methanesulfonic anhydride and N-alkylation by reductive amination using a ketone or an aldehyde in the presence of a reducing agent such as sodiumtriacetoxyborohydride.

Pharmaceutically acceptable salts may be prepared conventionally by reaction with the appropriate acid or acid derivative.

The compounds of the present invention inhibit the GlyT1 transporter. The compounds may selectively inhibit the GlyT1 transporter over the GlyT2 transporter.

Such compounds would be suitable for the treatment of certain neurological and neuropsychiatric disorders. As used herein, the terms "treatment" and "treating" refer to the alleviation and/or cure of established symptoms as well as prophylaxis.

The affinities of the compounds of this invention for the GlyT1 transporter can be determined by the following assay:
HEK293 cells expressing the Glycine (Type 1) transporter were grown in cell culture medium [DMEM/NUT mix F12 containing 2mM L-Glutamine, 0.8mg/mL G418 and 10% heat inactivated fetal calf serum] at 37°C and 5% CO₂. Cells grown to 70-80% confluency in T175 flasks were harvested and resuspended at 1.32x10⁶ cells/mL in assay buffer [140mM NaCl, 5.4mM KCI, 1.8mM CaCl₂, 0.8mM MgSO₄, 20mM HEPES, 5mM glucose and 5mM alanine, pH 7.4]. Compounds were serially diluted 2.5-fold in DMSO from a top concentration of 2.5mM with each compound giving a 11 data point dose-response. 100nL of compound at each concentration was added to the assay plate. An equal volume of Leadseeker™ WGA SPA beads (12.5mg/ml suspended in assay buffer) was added to the cell suspension (1.32 x 10⁶) and 5µL of the cell/bead suspension transferred to each well of a 384-well white solid bottom plate (3300 cells/well) containing 100nL of test compounds. Substrate (5µL) was added to each well [1:100 dilution of [³H]-glycine stock in assay buffer containing 2.5µM glycine). Final DMSO concentration was 1% v/v. Data was collected using a Perkin Elmer Viewlux. pIC₅₀ values were determined using ActivityBase.

The following assay may also be used:
HEK293 cells expressing the Glycine (Type 1) transporter are grown in cell medium (DMEM/NUT mix F12) containing 2 mM L-Glutamine, 0.8 mg/mL G418 and 10% heat inactivated fetal calf serum (Gibco BRL) at 37°C in 5% CO₂. Cells grown to 70-80% confluency in T175 flasks are harvested and resuspended at 4x10⁵ cells/ml in assay buffer [NaCl (140 mM), KCI (5.4 mM), CaCl₂ (1.8 mM), MgSO₄ (0.8 mM), HEPES (20mM), glucose (5 mM) and alanine (5 mM), pH 7.4]. An equal volume of Leadseeker™ SPA beads (12.5mg/ml suspended in assay buffer) is added to the cell suspension. Compounds are prepared as 10mM stocks in DMSO. 2.5 fold serial dilutions of the compounds are made in DMSO from a top conc of 2.5 mM. 100 nL of compound at each concentration is added to the assay plate (384-well white solid bottom plate) using the hummingbird dispenser. 5uL of the cell/bead mix is then added on top of the compound using a multidrop dispenser. Substrate (5uL) is then added to each well (1:100 dilution of H3-glycine in assay buffer containing 2.5 uM glycine) Data is collected using a PerkinElmer Viewlux as 5 minute exposures. pIC50 data values are determined using Activity Base.

Compounds may be assayed in their free base form or in the form of a salt, for example the hydrochloride salt or the formate salt. The assays described above are generally considered to provide data that is correct to ±3 standard deviations = ±0.5.

Compounds having a pIC₅₀ at the GlyT1 transporter of greater than or equal to 5.0 are considered to be active at the GlyT1 transporter. The example compounds below were found to have a pIC₅₀ at the GlyT1 transporter of greater than or equal to 5.0.

Accordingly, in a further aspect of the invention, there is provided a compound of formula (I) or a salt or solvate thereof: for use in therapy.

In another aspect of the invention, there is provided a compound of formula (I) as hereinbefore described or a salt or solvate thereof, for use in the treatment of a disorder mediated by GlyT1.

As used herein, the term "a disorder mediated by GlyT1" refers to a disorder that may be treated by the administration of a medicament that alters the activity of the GlyT1 transporter. As hereinbefore described, the action of GlyT1 transporters affects the local concentration of glycine around NMDA receptors. As a certain amount of glycine is needed for the efficient functioning of NMDA receptors, any change to that local concentration can affect NMDA-mediated neurotransmission. As hereinbefore described, changes in NMDA-mediated neurotransmission have been implicated in certain neuropsychiatric disorders such as dementia, depression and psychoses, for example schizophrenia, and learning and memory disorders, for example attention deficit disorders and autism. Thus, alterations in the activity of the GlyT1 transporter are expected to influence such disorders.

The disorders mediated by GlyT1 referred to herein include neurological and neuropsychiatric disorders, including psychoses such as schizophrenia, dementia and other forms of impaired cognition such as attention deficit disorders and organic brain syndromes. Other neuropsychiatric disorders include drug-induced (phencyclidine, ketamine and other dissociative anesthetics, amphetamine and other psychostimulants and cocaine) psychosis, psychosis associated with affective disorders, brief reactive psychosis, schizoaffective psychosis, and psychosis NOS, "schizophrenia-spectrum" disorders such as schizoid or schizotypal personality disorders, or illness associated with psychosis (such as major depression, manic depressive (bipolar) disorder, Alzheimer's disease and post-traumatic stress syndrome), and NMDA receptor-related disorders such as autism, depression, benign forgetfulness, childhood learning disorders and closed head injury.

The compounds of formula (I) are of use as antipsychotic agents for example in the treatment of schizophrenia, schizo-affective disorders, schizophreniform diseases, psychotic depression, mania, acute mania, paranoid and delusional disorders.

Within the context of the present invention, the terms used herein are classified in the Diagnostic and Statistical Manual of Mental Disorders, 4th Edition, published by the American Psychiatric Association (DSM-IV) and/or the International Classification of Diseases, 10^{th} Edition (ICD-10). The various subtypes of the disorders mentioned herein are contemplated as part of the present invention. Numbers in brackets after the listed diseases below refer to the classification code in DSM-IV.

In particular, the compounds of formula (I) are of use in the treatment of schizophrenia including the subtypes Paranoid Type (295.30), Disorganised Type (295.10), Catatonic Type (295.20), Undifferentiated Type (295.90) and Residual Type (295.60); Schizophreniform Disorder (295.40); Schizoaffective Disorder (295.70) including the subtypes Bipolar Type and Depressive Type; Delusional Disorder (297.1) including the subtypes Erotomanic Type, Grandiose Type, Jealous Type, Persecutory Type, Somatic Type, Mixed Type and Unspecified Type; Brief Psychotic Disorder (298.8); Shared Psychotic Disorder (297.3); Psychotic Disorder Due to a General Medical Condition including the subtypes With Delusions and With Hallucinations; Substance-Induced Psychotic Disorder including the subtypes With Delusions (293.81) and With Hallucinations (293.82); and Psychotic Disorder Not Otherwise Specified (298.9).

The compounds of formula (I) are also of use in the treatment of mood disorders including Major Depressive Episode, Manic Episode, Mixed Episode and Hypomanic Episode; Depressive Disorders including Major Depressive Disorder, Dysthymic Disorder (300.4), Depressive Disorder Not Otherwise Specified (311); Bipolar Disorders including Bipolar I Disorder, Bipolar II Disorder (Recurrent Major Depressive Episodes with Hypomanic Episodes) (296.89), Cyclothymic Disorder (301.13) and Bipolar Disorder Not Otherwise Specified (296.80); Other Mood Disorders including Mood Disorder Due to a General Medical Condition (293.83) which includes the subtypes With Depressive Features, With Major Depressive-like Episode, With Manic Features and With Mixed Features), Substance-Induced Mood Disorder (including the subtypes With Depressive Features, With Manic Features and With Mixed Features) and Mood Disorder Not Otherwise Specified (296.90).

The compounds of formula (I) are also of use in the treatment of anxiety disorders including Panic Attack, Agoraphobia, Panic Disorder, Agoraphobia Without History of Panic Disorder (300.22), Specific Phobia (300.29) including the subtypes Animal Type, Natural Environment Type, Blood-Injection-Injury Type, Situational Type and Other Type), Social Phobia (300.23), Obsessive-Compulsive Disorder (300.3), Posttraumatic Stress Disorder (309.81), Acute Stress Disorder (308.3), Generalized Anxiety Disorder (300.02), Anxiety Disorder Due to a General Medical Condition (293.84), Substance-Induced Anxiety Disorder and Anxiety Disorder Not Otherwise Specified (300.00).

The compounds of formula (I) are also of use in the treatment of substance-related disorders including Substance Use Disorders such as Substance Dependence and Substance Abuse; Substance-Induced Disorders such as Substance Intoxication, Substance Withdrawal, Substance-Induced Delirium, Substance-Induced Persisting Dementia, Substance-Induced Persisting Amnestic Disorder, Substance-Induced Psychotic Disorder, Substance-Induced Mood Disorder, Substance-Induced Anxiety Disorder, Substance-Induced Sexual Dysfunction, Substance-Induced Sleep Disorder and Hallucinogen Persisting Perception Disorder (Flashbacks); Alcohol-Related Disorders such as Alcohol Dependence (303.90), Alcohol Abuse (305.00), Alcohol Intoxication (303.00), Alcohol Withdrawal (291.81), Alcohol Intoxication Delirium, Alcohol Withdrawal Delirium, Alcohol-Induced Persisting Dementia, Alcohol-Induced Persisting Amnestic Disorder, Alcohol-Induced Psychotic Disorder, Alcohol-Induced Mood Disorder, Alcohol-Induced Anxiety Disorder, Alcohol-Induced Sexual Dysfunction, Alcohol-Induced Sleep Disorder and Alcohol-Related Disorder Not Otherwise Specified (291.9); Amphetamine (or Amphetamine-Like)-Related Disorders such as Amphetamine Dependence (304.40), Amphetamine Abuse (305.70), Amphetamine Intoxication (292.89), Amphetamine Withdrawal (292.0), Amphetamine Intoxication Delirium, Amphetamine Induced Psychotic Disorder, Amphetamine-Induced Mood Disorder, Amphetamine-Induced Anxiety Disorder, Amphetamine-Induced Sexual Dysfunction, Amphetamine-Induced Sleep Disorder and Amphetamine-Related Disorder Not Otherwise Specified (292.9); Caffeine Related Disorders such as Caffeine Intoxication (305.90), Caffeine-Induced Anxiety Disorder, Caffeine-Induced Sleep Disorder and Caffeine-Related Disorder Not Otherwise Specified (292.9); Cannabis-Related Disorders such as Cannabis Dependence (304.30), Cannabis Abuse (305.20), Cannabis Intoxication (292.89), Cannabis Intoxication Delirium, Cannabis-Induced Psychotic Disorder, Cannabis-Induced Anxiety Disorder and Cannabis-Related Disorder Not Otherwise Specified (292.9); Cocaine-Related Disorders such as Cocaine Dependence (304.20), Cocaine Abuse (305.60), Cocaine Intoxication (292.89), Cocaine Withdrawal (292.0), Cocaine Intoxication Delirium, Cocaine-Induced Psychotic Disorder, Cocaine-Induced Mood Disorder, Cocaine-Induced Anxiety Disorder, Cocaine-Induced Sexual Dysfunction, Cocaine-Induced Sleep Disorder and Cocaine-Related Disorder Not Otherwise Specified (292.9); Hallucinogen-Related Disorders such as Hallucinogen Dependence (304.50), Hallucinogen Abuse (305.30), Hallucinogen Intoxication (292.89), Hallucinogen Persisting Perception Disorder (Flashbacks) (292.89), Hallucinogen Intoxication Delirium, Hallucinogen-Induced Psychotic Disorder, Hallucinogen-Induced Mood Disorder, Hallucinogen-Induced Anxiety Disorder and Hallucinogen-Related Disorder Not Otherwise Specified (292.9); Inhalant-Related Disorders such as Inhalant Dependence (304.60), Inhalant Abuse (305.90), Inhalant Intoxication (292.89), Inhalant Intoxication Delirium, Inhalant-Induced Persisting Dementia, Inhalant-Induced Psychotic Disorder, Inhalant-Induced Mood Disorder, Inhalant-Induced Anxiety Disorder and Inhalant-Related Disorder Not Otherwise Specified (292.9); Nicotine-Related Disorders such as Nicotine Dependence (305.1), Nicotine Withdrawal (292.0) and Nicotine-Related Disorder Not Otherwise Specified (292.9); Opioid-Related Disorders such as Opioid Dependence (304.00), Opioid Abuse (305.50), Opioid Intoxication (292.89), Opioid Withdrawal (292.0), Opioid Intoxication Delirium, Opioid-Induced Psychotic Disorder, Opioid-Induced Mood Disorder, Opioid-Induced Sexual Dysfunction, Opioid-Induced Sleep Disorder and Opioid-Related Disorder Not Otherwise Specified (292.9); Phencyclidine (or Phencyclidine-Like)-Related Disorders such as Phencyclidine Dependence (304.60), Phencyclidine Abuse (305.90), Phencyclidine Intoxication (292.89), Phencyclidine Intoxication Delirium, Phencyclidine-Induced Psychotic Disorder, Phencyclidine-Induced Mood Disorder, Phencyclidine-Induced Anxiety Disorder and Phencyclidine-Related Disorder Not Otherwise Specified (292.9); Sedative-, Hypnotic-, or Anxiolytic-Related Disorders such as Sedative, Hypnotic, or Anxiolytic Dependence (304.10), Sedative, Hypnotic, or Anxiolytic Abuse (305.40), Sedative, Hypnotic, or Anxiolytic Intoxication (292.89), Sedative, Hypnotic, or Anxiolytic Withdrawal (292.0), Sedative, Hypnotic, or Anxiolytic Intoxication Delirium, Sedative, Hypnotic, or Anxiolytic Withdrawal Delirium, Sedative-, Hypnotic-, or Anxiolytic-Persisting Dementia, Sedative-, Hypnotic-, or Anxiolytic- Persisting Amnestic Disorder, Sedative-, Hypnotic-, or Anxiolytic-Induced Psychotic Disorder, Sedative-, Hypnotic-, or Anxiolytic-Induced Mood Disorder, Sedative-, Hypnotic-, or Anxiolytic-Induced Anxiety Disorder Sedative-, Hypnotic-, or Anxiolytic-Induced Sexual Dysfunction, Sedative-, Hypnotic-, or Anxiolytic-Induced Sleep Disorder and Sedative-, Hypnotic-, or Anxiolytic-Related Disorder Not Otherwise Specified (292.9); Polysubstance-Related Disorder such as Polysubstance Dependence (304.80); and Other (or Unknown) Substance-Related Disorders such as Anabolic Steroids, Nitrate Inhalants and Nitrous Oxide.

The compounds of formula (I) are also of use in the treatment of sleep disorders including primary sleep disorders such as Dyssomnias such as Primary Insomnia (307.42), Primary Hypersomnia (307.44), Narcolepsy (347), Breathing-Related Sleep Disorders (780.59), Circadian Rhythm Sleep Disorder (307.45) and Dyssomnia Not Otherwise Specified (307.47); primary sleep disorders such as Parasomnias such as Nightmare Disorder (307.47), Sleep Terror Disorder (307.46), Sleepwalking Disorder (307.46) and Parasomnia Not Otherwise Specified (307.47); Sleep Disorders Related to Another Mental Disorder such as Insomnia Related to Another Mental Disorder (307.42) and Hypersomnia Related to Another Mental Disorder (307.44); Sleep Disorder Due to a General Medical Condition; and Substance-Induced Sleep Disorder including the subtypes Insomnia Type, Hypersomnia Type, Parasomnia Type and Mixed Type.

The compounds of formula (I) are also of use in the treatment of eating disorders such as Anorexia Nervosa (307.1) including the subtypes Restricting Type and Binge-Eating/Purging Type; Bulimia Nervosa (307.51) including the subtypes Purging Type and Nonpurging Type; Obesity; Compulsive Eating Disorder; and Eating Disorder Not Otherwise Specified (307.50).

The compounds of formula (I) are also of use in the treatment of Autistic Disorder (299.00); Attention-Deficit /Hyperactivity Disorder including the subtypes Attention-Deficit /Hyperactivity Disorder Combined Type (314.01), Attention-Deficit /Hyperactivity Disorder Predominantly Inattentive Type (314.00), Attention-Deficit /Hyperactivity Disorder Hyperactive-Impulse Type (314.01) and Attention-Deficit /Hyperactivity Disorder Not Otherwise Specified (314.9); Hyperkinetic Disorder; Disruptive Behaviour Disorders such as Conduct Disorder including the subtypes childhood-onset type (321.81), Adolescent-Onset Type (312.82) and Unspecified Onset (312.89), Oppositional Defiant Disorder (313.81) and Disruptive Behaviour Disorder Not Otherwise Specified; and Tic Disorders such as Tourette's Disorder (307.23).

The compounds of formula (I) are also of use in the treatment of Personality Disorders including the subtypes Paranoid Personality Disorder (301.0), Schizoid Personality Disorder (301.20), Schizotypal Personality Disorder (301,22), Antisocial Personality Disorder (301.7), Borderline Personality Disorder (301,83), Histrionic Personality Disorder (301.50), Narcissistic Personality Disorder (301,81), Avoidant Personality Disorder (301.82), Dependent Personality Disorder (301.6), Obsessive-Compulsive Personality Disorder (301.4) and Personality Disorder Not Otherwise Specified (301.9).

The compounds of Formula (I) are also of use in the enhancement of cognition including the treatment of cognition impairment in other diseases such as schizophrenia, bipolar disorder, depression, other psychiatric disorders and psychotic conditions associated with cognitive impairment. Within the context of the present invention, the term cognitive impairment includes for example the treatment of impairment of cognitive functions including attention, orientation, learning disorders, memory (i.e. memory disorders, amnesia, amnesic disorders, transient global amnesia syndrome and age-associated memory impairment) and language function; cognitive impairment as a result of stroke, Alzheimer's disease, Huntington's disease, Pick disease, Aids-related dementia or other dementia states such as Multiinfarct dementia, alcoholic dementia, hypotiroidism-related dementia, and dementia associated to other degenerative disorders such as cerebellar atrophy and amyotropic lateral sclerosis; other acute or sub-acute conditions that may cause cognitive decline such as delirium or depression (pseudodementia states) trauma, head trauma, age related cognitive decline, stroke, neurodegeneration, drug-induced states, neurotoxic agents, mild cognitive impairment, age related cognitive impairment, autism related cognitive impairment, Down's syndrome, cognitive deficit related to psychosis, and post-electroconvulsive treatment related cognitive disorders; and dyskinetic disorders such as Parkinson's disease, neuroleptic-induced parkinsonism, and tardive dyskinesias.

The compounds of formula (I) are also of use in the treatment of sexual dysfunctions including Sexual Desire Disorders such as Hypoactive Sexual Desire Disorder (302.71), and Sexual Aversion Disorder (302.79); sexual arousal disorders such as Female Sexual Arousal Disorder (302.72) and Male Erectile Disorder (302.72); orgasmic disorders such as Female Orgasmic Disorder (302.73), Male Orgasmic Disorder (302.74) and Premature Ejaculation (302.75); sexual pain disorder such as Dyspareunia (302.76) and Vaginismus (306.51); Sexual Dysfunction Not Otherwise Specified (302.70); paraphilias such as Exhibitionism (302.4), Fetishism (302.81), Frotteurism (302.89), Pedophilia (302.2), Sexual Masochism (302.83), Sexual Sadism (302.84), Transvestic Fetishism (302.3), Voyeurism (302.82) and Paraphilia Not Otherwise Specified (302.9); gender identity disorders such as Gender Identity Disorder in Children (302.6) and Gender Identity Disorder in Adolescents or Adults (302.85); and Sexual Disorder Not Otherwise Specified (302.9).

The invention also provides a compound of formula (I) as hereinbefore described or a pharmaceutically acceptable salt or solvate thereof for use in the treatment of schizophrenia, mood disorders, anxiety disorders, substance-related disorders, sleep disorders, eating disorders, autistic disorder, attention-deficit/hyperactivity disorder, disruptive behaviour disorder, tic disorders, personality disorders, cognition impairment in other diseases, sexual dysfunction, Parkinson's disease, dyskinetic disorders, depression, bipolar disorder, cognitive impairment, obesity, emesis, movement disorders, obsessive-compulsive disorders, amnesia, aggression, vertigo, dementia and circadian rhythm disorders.

The invention also provides a compound of formula (I) as hereinbefore described or a pharmaceutically acceptable salt or solvate thereof for use in the treatment of psychotic disorders, schizophrenia, Parkinson's disease, substance abuse, dyskinetic disorders, depression, bipolar disorder, anxiety, cognitive impairment, eating disorders, obesity, sexual dysfunction, sleep disorders, emesis, movement disorders, obsessive-compulsive disorders, amnesia, aggression, autism, vertigo, dementia, circadian rhythm disorders and gastric motility disorders.

In another aspect of the invention, there is provided a method of treating a mammal, including a human, suffering from or susceptible to a disorder mediated by GlyT1, which comprises administering an effective amount of a compound of formula (I) as hereinbefore defined or a salt or solvate thereof.

The invention also provides a method of treating schizophrenia, mood disorders, anxiety disorders, substance-related disorders, sleep disorders, eating disorders, autistic disorder, attention-deficit/hyperactivity disorder, disruptive behaviour disorder, tic disorders, personality disorders, cognition impairment in other diseases, sexual dysfunction, Parkinson's disease, dyskinetic disorders, depression, bipolar disorder, cognitive impairment, obesity, emesis, movement disorders, obsessive-compulsive disorders, amnesia, aggression, vertigo, dementia and circadian rhythm disorders which comprises administering to a mammal in need thereof an effective amount of a compound of formula (I) as hereinbefore described or a pharmaceutically acceptable salt or solvate thereof.

The invention also provides a method of treating psychotic disorders, schizophrenia, Parkinson's disease, substance abuse, dyskinetic disorders, depression, bipolar disorder, anxiety, cognitive impairment, eating disorders, obesity, sexual dysfunction, sleep disorders, emesis, movement disorders, obsessive-compulsive disorders, amnesia, aggression, autism, vertigo, dementia, circadian rhythm disorders and gastric motility disorders which comprises administering to a mammal in need thereof an effective amount of a compound of formula (I) as hereinbefore described or a pharmaceutically acceptable salt or solvate thereof.

The compounds of formula (I) are also of use as anticonvulsants. The compounds of formula (I) are thus useful in the treatment of convulsions in mammals, and particularly epilepsy in humans. "Epilepsy" is intended to include the following seizures: simple partial seizures, complex partial seizures, secondary generalised seizures, generalised seizures including absence seizures, myoclonic seizures, clonic seizures, tonic seizures, tonic clonic seizures and atonic seizures. The invention also provides a method of treating convulsions, which comprises administering to a mammal in need thereof an effective amount of a compound of formula (I) as hereinbefore described or a pharmaceutically acceptable salt or solvate thereof. Treatment of epilepsy may be carried out by the administration of a non-toxic anticonvulsant effective amount of a compound of the formula (III) or a pharmaceutically acceptable salt, or a composition as hereinbefore defined.

The compounds of formula (I) also find use in the treatment of neuropathic pain, for example in diabetic neuropathy, sciatica, non-specific lower back pain, multiple sclerosis pain, fibromyalgia, HIV-related neuropathy, neuralgia such as post-herpetic neuralgia and trigeminal neuralgia and pain resulting from physical trauma, amputation, cancer, toxins or chronic inflammatory conditions.

In another aspect of the invention, there is provided use of a compound of formula (I) as hereinbefore defined or a salt or solvate thereof in the preparation of a medicament for the treatment of a disorder mediated by GlyT1.

Preferably, the disorder mediated by GlyT1 to be treated by the use or method as hereinbefore described is a psychosis, including schizophrenia, dementia and attention deficit disorders, particularly schizophrenia.

The invention also provides the use of a compound of formula (I) as hereinbefore described or a pharmaceutically acceptable salt or solvate thereof in the manufacture of a medicament for the treatment of schizophrenia, mood disorders, anxiety disorders, substance-related disorders, sleep disorders, eating disorders, autistic disorder, attention-deficit/hyperactivity disorder, disruptive behaviour disorder, tic disorders, personality disorders, cognition impairment in other diseases, sexual dysfunction, Parkinson's disease, dyskinetic disorders, depression, bipolar disorder, cognitive impairment, obesity, emesis, movement disorders, obsessive-compulsive disorders, amnesia, aggression, vertigo, dementia and circadian rhythm disorders.

The invention also provides the use of a compound of formula (I) as hereinbefore described or a pharmaceutically acceptable salt or solvate thereof in the manufacture of a medicament for the treatment of psychotic disorders, schizophrenia, Parkinson's disease, substance abuse, dyskinetic disorders, depression, bipolar disorder, anxiety, cognitive impairment, eating disorders, obesity, sexual dysfunction, sleep disorders, emesis, movement disorders, obsessive-compulsive disorders, amnesia, aggression, autism, vertigo, dementia, circadian rhythm disorders and gastric motility disorders.

As used herein, the term "effective amount" means that amount of a drug or pharmaceutical agent that will elicit the biological or medical response of a tissue, system, animal or human that is being sought, for instance, by a researcher or clinician.

Compounds for use according to the invention may be administered as the raw material but the active ingredients are preferably provided in the form of pharmaceutical compositions.

Accordingly, in a further aspect of the invention, there is provided a pharmaceutical composition comprising a compound of formula (I) as hereinbefore described or a salt or solvate thereof, and at least one pharmaceutically acceptable carrier, diluent or excipient.

These pharmaceutical compositions may be used in the treatment of clinical conditions for which a GlyT1 inhibitor is indicated such as, for example, schizophrenia. The carrier must be pharmaceutically acceptable to the recipient and must be compatible with, i.e. not have a deleterious effect upon, the other ingredients in the composition. The carrier may be a solid or a liquid and is preferably formulated with at least one compound of formula (I) or a salt or solvate thereof as a unit dose formulation. If desired, other physiologically active ingredients may also be incorporated in the pharmaceutical compositions of the invention.

It will be appreciated by those skilled in the art that the compounds according to the invention may advantageously be used in conjunction with one or more other therapeutic agents, for instance, different antidepressant agents such as 5HT3 antagonists, serotonin agonists, NK-1 antagonists, selective serotonin reuptake inhibitors (SSRI), noradrenaline re-uptake inhibitors (SNRI), tricyclic antidepressants, dopaminergic antidepressants, H3 antagonists, 5HT1A antagonists, 5HT1B antagonists, 5HT1D antagonists, D1 agonists, M1 agonists and/or anticonvulsant agents, as well as atypical antipsychotic drugs and cognitive enhancers.

Suitable 5HT3 antagonists which may be used in combination of the compounds of the inventions include for example ondansetron, granisetron, metoclopramide.

Suitable serotonin agonists which may be used in combination with the compounds of the invention include sumatriptan, rauwolscine, yohimbine, metoclopramide.

Suitable SSRIs which may be used in combination with the compounds of the invention include fluoxetine, citalopram, femoxetine, fluvoxamine, paroxetine, indalpine, sertraline, zimeldine.

Suitable SNRIs which may be used in combination with the compounds of the invention include venlafaxine and reboxetine.

Suitable tricyclic antidepressants which may be used in combination with a compound of the invention include imipramine, amitriptiline, chlomipramine and nortriptiline.

Suitable dopaminergic antidepressants which may be used in combination with a compound of the invention include bupropion and amineptine.

Suitable anticonvulsant agents which may be used in combination of the compounds of the invention include for example divalproex, carbamazepine and diazepam.

Suitable atypical antipsychotic drugs which which may be used in combination of the compounds of the invention include for example risperidone, olanzapine, ziprasidone, aripiprazole and clozapine.

It will be appreciated that the compounds of the combination or composition may be administered simultaneously (either in the same or different pharmaceutical formulations), separately or sequentially.

The compounds of formula (I) and their pharmaceutically acceptable salts and solvates thereof are also suitable for combination with other typical and atypical antipsychotics to provide improved treatment of psychotic disorders. Particular advantages associated with the combinations, uses and methods of treatment of compounds of formula (I) and their pharmaceutically acceptable salts and solvates thereof include equivalent or improved efficacy at doses of administration which are lower than those commonly used for the individual components. Improved treatments of positive symptoms and/or negative symptoms and/or cognitive symptoms of the psychotic disorder may also be observed. The combinations, uses and methods of treatment of the invention may also provide advantages in treatment of patients who fail to respond adequately or who are resistant to treatment with certain neuroleptic agents.

The combination therapies of the invention are preferably administered adjunctively. By adjunctive administration is meant the coterminous or overlapping administration of each of the components in the form of separate pharmaceutical compositions or devices. This regime of therapeutic administration of two or more therapeutic agents is referred to generally by those skilled in the art and herein as adjunctive therapeutic administration; it is also known as add-on therapeutic administration. Any and all treatment regimes in which a patient receives separate but coterminous or overlapping therapeutic administration of the compounds of formula (I) or a pharmaceutically acceptable salt or solvate thereof and at least one neuroleptic agent are within the scope of the current invention. In one embodiment of adjunctive therapeutic administration as described herein, a patient is typically stabilised on a therapeutic administration of one or more of the components for a period of time and then receives administration of another component. Within the scope of this invention, it is preferred that the compounds of formula (I) or a pharmaceutically acceptable salt or solvate thereof is administered as adjunctive therapeutic treatment to patients who are receiving administration of at least one neuroleptic agent, but the scope of the invention also includes the adjunctive therapeutic administration of at least one neuroleptic agent to patients who are receiving administration of compounds of formula (I) or a pharmaceutically acceptable salt or solvate thereof.

The combination therapies of the invention may also be administered simultaneously. By simultaneous administration is meant a treatment regime wherein the individual components are administered together, either in the form of a single pharmaceutical composition or device comprising or containing both components, or as separate compositions or devices, each comprising one of the components, administered simultaneously. Such combinations of the separate individual components for simultaneous combination may be provided in the form of a kit-of-parts.

In a further aspect therefore, the invention provides a method of treatment of a psychotic disorder by adjunctive therapeutic administration of compounds of formula (I) or a pharmaceutically acceptable salt or solvate thereof to a patient receiving therapeutic administration of at least one neuroleptic agent. In a further aspect, the invention provides the use of compounds of formula (I) or a pharmaceutically acceptable salt or solvate thereof in the manufacture of a medicament for adjunctive therapeutic administration for the treatment of a psychotic disorder in a patient receiving therapeutic administration of at least one neuroleptic agent. The invention further provides compounds of formula (I) or a pharmaceutically acceptable salt or solvate thereof for use for adjunctive therapeutic administration for the treatment of a psychotic disorder in a patient receiving therapeutic administration of at least one neuroleptic agent.

In a further aspect, the invention provides a method of treatment of a psychotic disorder by adjunctive therapeutic administration of at least one neuroleptic agent to a patient receiving therapeutic administration of compounds of formula (I) or a pharmaceutically acceptable salt or solvate thereof. In a further aspect, the invention provides the use of at least one neuroleptic agent in the manufacture of a medicament for adjunctive therapeutic administration for the treatment of a psychotic disorder in a patient receiving therapeutic administration of compounds of formula (I) or a pharmaceutically acceptable salt or solvate thereof. The invention further provides at least one neuroleptic agent for adjunctive therapeutic administration for the treatment of a psychotic disorder in a patient receiving therapeutic administration of compounds of formula (I) or a pharmaceutically acceptable salt or solvate thereof.

In a further aspect, the invention provides a method of treatment of a psychotic disorder by simultaneous therapeutic administration of compounds of formula (I) or a pharmaceutically acceptable salt or solvate thereof in combination with at least one neuroleptic agent. The invention further provides the use of a combination of compounds of formula (I) or a pharmaceutically acceptable salt or solvate thereof and at least one neuroleptic agent in the manufacture of a medicament for simultaneous therapeutic administration in the treatment of a psychotic disorder. The invention further provides the use of compounds of formula (I) or a pharmaceutically acceptable salt thereof in the manufacture of a medicament for simultaneous therapeutic administration with at least one neuroleptic agent in the treatment of a psychotic disorder. The invention further provides compounds of formula (I) or a pharmaceutically acceptable salt thereof for use for simultaneous therapeutic administration with at least one neuroleptic agent in the treatment of a psychotic disorder. The invention further provides the use of at least one neuroleptic agent in the manufacture of a medicament for simultaneous therapeutic administration with compounds of formula (I) or a pharmaceutically acceptable salt thereof in the treatment of a psychotic disorder.

In further aspects, the invention provides a method of treatment of a psychotic disorder by simultaneous therapeutic administration of a pharmaceutical composition comprising compounds of formula (I) or a pharmaceutically acceptable salt or solvate thereof and at least one mood stabilising or antimanic agent, a pharmaceutical composition comprising compounds of formula (I) or a pharmaceutically acceptable salt or solvate thereof and at least one mood stabilising or antimanic agent, the use of a pharmaceutical composition comprising compounds of formula (I) or a pharmaceutically acceptable salt or solvate thereof and at least one mood stabilising or antimanic agent in the manufacture of a medicament for the treatment of a psychotic disorder, and a pharmaceutical composition comprising compounds of formula (I) or a pharmaceutically acceptable salt or solvate thereof and at least one mood stabilising or antimanic agent for use in the treatment of a psychotic disorder.

In a further aspect, the invention provides a kit-of-parts for use in the treatment of a psychotic disorder comprising a first dosage form comprising compounds of formula (I) or a pharmaceutically acceptable salt or solvate thereof and one or more further dosage forms each comprising a neuroleptic agent for simultaneous therapeutic administration.

Within the context of the present invention, the term psychotic disorder includes those disorders mentioned above, such as schizophrenia, mood disorders, anxiety disorders, substance-related disorders, sleep disorders, eating disorders, autistic disorder, attention-deficit/hyperactivity disorder, disruptive behaviour disorder, tic disorders, personality disorders, cognition impairment in other diseases, sexual dysfunction, dyskinetic disorders, depression, bipolar disorder, cognitive impairment and obsessive-compulsive disorders and all the various forms of the disorders as mentioned herein. which are contemplated as part of the present invention.

Examples of neuroleptic/antipsychotic drugs that are useful in the present invention include, but are not limited to: butyrophenones, such as haloperidol, pimozide, and droperidol; phenothiazines, such as chlorpromazine, thioridazine, mesoridazine, trifluoperazine, perphenazine, fluphenazine, thiflupromazine, prochlorperazine, and acetophenazine; thioxanthenes, such as thiothixene and chlorprothixene ; thienobenzodiazepines; dibenzodiazepines; benzisoxazoles; dibenzothiazepines; imidazolidinones ; benzisothiazolyl-piperazines; triazine such as lamotrigine; dibenzoxazepines, such as loxapine; dihydroindolones, such as molindone; aripiprazole; and derivatives thereof that have antipsychotic activity.

Examples of neuroleptic drugs that are preferred for use in the present invention are shown in Table A.

**Table A**

| Neuroleptic drugs | | | | |
|---|---|---|---|---|
| **Common Name** | **Trade Name** | **Route of Administration** | **Form** | **Dosage Range and (Median)^{a}** |
| Clozapine | **CLOZARIL** | oral | tablets | 12.5-900 mg/day |
| | | | | (300-900 mg/day) |
| Olanzapine | **ZYPREXA** | oral | tablets | 5-25 mg/day **(10-25 mg/day)** |
| Ziprasidone | **GEODON** | oral | capsules | **20-80mg/twice a day (80-160 mg/day)** |
| Risperidone | **RISPERDAL** | oral | solution tablets | **2-16 mg/day tablets (4-12 mg/day)** |
| Quetiapine fumarate | **SEROQUEL** | oral | tablets | **50-900 mg/day (300-900 mg/day)** |
| Sertindole | **SERLECT** | | | **(4-24 mg/day)** |
| Amisulpride | | | | |
| Haloperidol | **HALDOL** | oral | tablets | **1-100 mg/day (1-15 mg/day)** |
| Haloperidol Decanoate | **HALDOL Decanoate** | parenteral | injection | |
| Haloperidol lactate | **HALDOL INTENSOL** | oral | solution | |
| | | parenteral | injection | |
| Chlorpromazine | **THORAZINE** | rectal | suppositories | 30-800 mg/day |
| | | oral | capsules solution tablets | (200-500 mg/day) |
| | | parenteral | injection | |
| Fluphenazine | PROLIXIN | | | **0.5-40 mg/day (1-5 mg/day)** |
| Fluphenazine decanoate | **PROLIXIN Decanoate** | parenteral | injection | **(about one-half the dosage shown for oral)** |
| Fluphenazine enanthate | **PROLIXIN** | parenteral | injection | **(same as above** |
| Fluphenazine hydrochloride | **PROLIXIN** | oral | elixer solution | |
| | | parenteral | injection | |
| Thiothixene | **NAVANE** | oral | capsules | **6-60 mg/day (8-30 mg/day)** |
| Thiothixene hydrochloride | **NAVANE** | oral | solution | |
| | | parenteral | injection | |
| Trifluoperazine | **STELAZINE** | | | **(2-40 mg/day)** |
| Perphenazine | **TRILAFON** | oral | solution tablets | 12-64 mg/day (16-64 mg/day) |
| | | parenteral | injection | |
| Perpehazine and Amitriptyline hydrochloride | **ETRAFON TRIAVIL** | oral | tablets | |
| Thioridazine | **MELLARIL** | oral | suspension solution tablets | **150-800 mg/day (100-300 mg/day)** |
| Mesoridazine | | | | **(30-400 mg/day)** |
| | | | | |
| Molindone | **MOBAN** | | | **50-225 mg/day (15-150 mg/day)** |
| Molindone hydrochloride | **MOBAN** | oral | solution | |
| Loxapine | **LOXITANE** | | | **20-250 mg/day (60-100 mg/dav)** |
| Loxapine hydrochloride | **LOXITANE** | oral | solution | |
| | | parenteral | injection | |
| Loxapine succinate | LOXITANE | oral | capsules | |
| Pimozide | | | | **(1-10 mg/day)** |
| Flupenthixol | | | | |
| Promazine | **SPARINE** | | | |
| Triflupromazine | **VESPRIN** | | | |
| Chlorprothixene | **TARACTAN** | | | |
| Droperidol | **INAPSINE** | | | |
| Acetophenazine | **TINDAL** | | | |
| Prochlorperazine | **COMPAZINE** | | | |
| Methotrimeprazine | **NOZINAN** | | | |
| Pipotiazine | **PIPOTRIL** | | | |
| Aripiprazole | | | | |
| Hoperidone | | | | |

Examples of tradenames and suppliers of selected neuroleptic drugs are as follows : clozapine (available under the tradename CLOZARIL®, from Mylan, Zenith Goldline, UDL, Novartis); olanzapine (available under the tradename ZYPREX®, from Lilly ; ziprasidone (available under the tradename GEODON®, from Pfizer); risperidone (available under the tradename RISPERDAL®, from Janssen); quetiapine fumarate (available under the tradename SEROQUEL®, from AstraZeneca); haloperidol (available under the tradename HALDOL®, from Ortho-McNeil); chlorpromazine (available under the tradename THORAZINE®, from SmithKline Beecham (GSK); fluphenazine (available under the tradename PROLIXIN®, from Apothecon, Copley, Schering, Teva, and American Pharmaceutical Partners, Pasadena); thiothixene (available under the tradename NAVANE®;, from Pfizer); trifluoperazine (10-[3-(4-methyl-1-piperazinyl)propyl]-2-(trifluoromethyl)phenothiazine dihydrochloride, available under the tradename STELAZINE®, from Smith Klein Beckman; perphenazine (available under the tradename TRILAFON®; from Schering); thioridazine (available under the tradename MELLARIL®; from Novartis, Roxane, HiTech, Teva, and Alpharma) ; molindone (available under the tradename MOBAN®, from Endo); and loxapine (available under the tradename LOXITANE®; from Watson). Furthermore, benperidol (Glianimon®), perazine (Taxilan®) or melperone (Eunerpan®)) may be used.

Other preferred neuroleptic drugs include promazine (available under the tradename SPARINE®), triflurpromazine (available under the tradename VESPRIN®), chlorprothixene (available under the tradename TARACTAN®), droperidol (available under the tradename INAPSINE®), acetophenazine (available under the tradename TINDAL®;), prochlorperazine (available under the tradename COMPAZINE®), methotrimeprazine (available under the tradename NOZINAN®), pipotiazine (available under the tradename PIPOTRIL®), ziprasidone, and hoperidone.

Particularly preferred neuroleptic agents for use in the invention are olanzapine, risperidone, quetiapine, aripiprazole, haloperidol, clozapine, ziprasidone and osanetant.

It will be appreciated by those skilled in the art that the compounds according to the invention may advantageously be used in conjunction with one or more other therapeutic agents, for instance, different antidepressant agents such as 5HT3 antagonists, serotonin agonists, NK-1 antagonists, selective serotonin reuptake inhibitors (SSRI), noradrenaline re-uptake inhibitors (SNRI), tricyclic antidepressants, dopaminergic antidepressants, H3 antagonists, 5HT1A antagonists, 5HT1B antagonists, 5HT1D antagonists, D1 agonists, M1 agonists and/or anticonvulsant agents, as well as atypical antipsychotic drugs and cognitive enhancers.

Suitable 5HT3 antagonists which may be used in combination of the compounds of the inventions include for example ondansetron, granisetron, metoclopramide.
Suitable serotonin agonists which may be used in combination with the compounds of the invention include sumatriptan, rauwolscine, yohimbine, metoclopramide.

Suitable SSRIs which may be used in combination with the compounds of the invention include fluoxetine, citalopram, femoxetine, fluvoxamine, paroxetine, indalpine, sertraline, zimeldine.

Suitable SNRIs which may be used in combination with the compounds of the invention include venlafaxine and reboxetine.

Suitable tricyclic antidepressants which may be used in combination with a compound of the invention include imipramine, amitriptiline, chlomipramine and nortriptiline.

Suitable dopaminergic antidepressants which may be used in combination with a compound of the invention include bupropion and amineptine.

Suitable anticonvulsant agents which may be used in combination of the compounds of the invention include for example divalproex, carbamazepine and diazepam.

Suitable atypical antipsychotic drugs which which may be used in combination of the compounds of the invention include for example risperidone, olanzapine, ziprasidone, aripiprazole and clozapine.

It will be appreciated that the compounds of the combination or composition may be administered simultaneously (either in the same or different pharmaceutical formulations), separately or sequentially.

For use in medicine, the compounds of the present invention are usually administered as a standard pharmaceutical composition. The present invention therefore provides in a further aspect a pharmaceutical composition comprising a compound of formula (I) as hereinbefore described or a pharmaceutically (i.e. physiologically) acceptable salt thereof and a pharmaceutically (i.e. physiologically) acceptable carrier. The pharmaceutical composition can be for use in the treatment of any of the conditions described herein.

Possible formulations include those suitable for oral, sub-lingual, buccal, parenteral (for example, subcutaneous, intramuscular, or intravenous), rectal, topical and intranasal administration and in forms suitable for administration by inhalation or insufflation (either through the mouth or nose). The most suitable means of administration for a particular patient will depend on the nature and severity of the conditions being treated and on the nature of the active compound, but, where possible, oral administration is preferred.

Formulations suitable for oral administration may be provided as discrete units, such as tablets, capsules, cachets, or lozenges, each containing a predetermined amount of the active compound; as powders or granules; as solutions or suspensions in aqueous or non-aqueous liquids; or as oil-in-water or water-in-oil emulsions. For example, a compound of the invention may be prepared as a formulation with a controlled release profile. This may be in any of the above mentioned pharmaceutical forms. For example, it may be a gel formulation in a non aqueous oily vehicle, for example Miglyol, with a suitable gelling agent if required, for example methyl cellulose or hydrophobic colloidal silica.

Formulations suitable for sublingual or buccal administration include lozenges comprising the active compound and, typically, a flavoured base, such as sugar and acacia or tragacanth and pastilles comprising the active compound in an inert base, such as gelatin and glycerin or sucrose and acacia.

Formulations suitable for parenteral administration typically comprise sterile aqueous solutions containing a predetermined concentration of the active compound; the solution is preferably isotonic with the blood of the intended recipient. Although such solutions are preferably administered intraveneously, they may also be administered by subcutaneous or intramuscular injection.

Formulations suitable for rectal administration are preferably provided as unit-dose suppositories comprising the active ingredient and one or more solid carriers forming the suppository base, for example, cocoa butter.

Formulations suitable for topical or intranasal application include ointments, creams, lotions, pastes, gels, sprays, aerosols and oils. Suitable carriers for such formulations include petroleum jelly, lanolin, polyethylene glycols, alcohols, and combinations thereof.

Formulations of compounds of the invention may, for example, be composed so as to improve the exposure profile of the compound of the invention.

Compositions suitable for transdermal administration include ointments, gels and patches. Preferably the composition is in unit dose form such as a tablet, capsule or ampoule.

The formulations of the invention may be prepared by any suitable method, typically by uniformly and intimately admixing the active compound(s) with liquids or finely divided solid carriers, or both, in the required proportions and then, if necessary, shaping the resulting mixture into the desired shape.

For example, a tablet may be prepared by compressing an intimate mixture comprising a powder or granules of the active ingredient and one or more optional ingredients, such as a binder, lubricant, inert diluent, or surface active dispersing agent, or by moulding an intimate mixture of powdered active ingredient and inert liquid diluent.

Aqueous solutions for parenteral administration are typically prepared by dissolving the active compound in sufficient water to give the desired concentration and then rendering the resulting solution sterile and isotonic.

It will be appreciated that the precise dose administered will depend on the age and condition of the patient and the frequency and route of administration and will be at the ultimate discretion of the attendant physician. The compound may be administered in single or divided doses and may be administered one or more times, for example 1 to 4 times per day.

A proposed dose of the active ingredient for use according to the invention for oral, sub-lingual, parenteral, buccal, rectal, intranasal or topical administration to a human (of approximately 70 kg bodyweight) for the treatment of neurological and neuropsychiatric disorders mediated by a GlyT1 inhibitor, including schizophrenia, may be about 1 to about 1000 mg, preferably about 5 to about 500 mg, more preferably about 10 to about 100 mg of the active ingredient per unit dose which could be administered, for example, 1 to 4 times per day.

All publications, including but not limited to patents and patent applications, cited in this specification are herein incorporated by reference as if each individual publication were specifically and individually indicated to be incorporated by reference herein as though fully set forth.

The invention is further illustrated by the following non-limiting examples.

### Examples

### Abbreviations:

- THF: tetrahydrofuran
- DCM: dichloromethane
- DMF: dimethylformamide
- EDC: N-(3-(dimethylamino)propyl)-N-ethylcarbodiimide hydrochloride
- HOAt: 3H-(1,2,3)-triazolo(4,5-b)pyridine-3-ol
- NMP: N-methylpyrrolidinone
- HOBt: 1-hydroxybenzotriazole hydrate
- DCC: dicyclohexylcarbodiimide

**Analytical LC/MS chromatography conditions:**

| | |
|---|---|
| Column: | Waters Atlantis 50mm x 4.6mm, 3um particle size |
| Mobile phase: | A: 0.05% Formic acid + Water |
| | B: Acetonitrile +0.05% Formic acid |
| Gradient: | 5-min runtime: 3%B to 97%B over 4min |
| Flow rate: | 3 ml/min |
| UV wavelength range: | 220 -330 nm |
| Temperature: | 30°C |

**Mass Directed Auto-Purification System chromatography conditions:**

| | |
|---|---|
| Column: | Waters Atlantis 19mm x 100mm or 30mm X 100mm, 5um |
| | particle size |
| Mobile phase: | A: 0.1% Formic acid + Water |
| | B: Acetonitrile +0.1 % Formic acid |
| Gradient: | 13.5 min runtime with 10min gradient dependant on |
| | analytical retention time |
| Flow rate: | 20 or 40 ml/min |

### General:

Throughout the examples section, the following terminology is adopted with regard to chiral compounds: when a mixture of two enantiomers has been prepared, the compound is described as (±). When a single enantiomer (that is to say mixture chirally enriched in one of the enantiomers) has been prepared, it is referred to as "chiral". The absolute stereochemistry has not been ascertained at the time of filing. Individual enantiomers of some materials prepared are identified by virtue of optical rotations and such materials are identified as the (+) or (-) enantiomers. Where optical rotation information is not yet available, individual enantiomers of the products are individually identifiable by virtue of the chiral HPLC characteristics of the amine intermediate.

Where reactions are described as having been carried out in a similar manner to earlier, more completely described reactions, the general reaction conditions used were essentially the same. Work up conditions used were of the types standard in the art, but may have been adapted from one reaction to another.

### Description 1: 1-(Dimethylamino)cyclopentanecarbonitrile

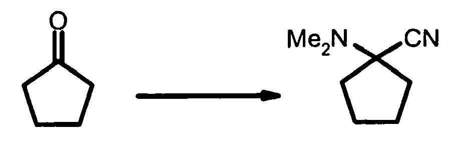

To a suspension of dimethylamine hydrochloride (8.15g; 0.1 mol) in cyclopentanone (8.4g; 0.1 mol) cooled (ice bath) was added dropwise a solution of potassium cyanide (6.5g; 0.1 mol) in water (50ml) over 10min. After vigorous stirring at room temperature for 18h, the crude reaction mixture was extracted three times with diethyl ether (3 x 200ml) and the combined extracts washed twice with water (2 x 50ml), dried (Na₂SO₄), and evaporated to afford the title product as a pale yellow oil (12.5g) which was used without further purification. ¹H NMR (CDCl₃) δ: 1.7 - 2.0 (6H, m), 2.15 - 2.3 (2H, m), 3.3 (6H, s). Mass Spectrum (Electrospray LC/MS): Found 112 (MH⁺-HCN). C₈H₁₄N₂ requires 138.

### Alternative method:

To a stirred, ice cooled mixture of dimethylamine hydrochloride (26.32.g; 0.323mol) and cyclopentanone (27.15g; 0.323mol) was added dropwise a solution of potassium cyanide (21.02g; 0.323mol) in water (170ml) over 10min. The mixture was stirred at room temperature overnight. Then, the mixture was extracted into diethylether (2 x 200ml) and the combined organics were washed with brine (200ml), dried (Na₂SO₄), and evaporated *in vacuo* to afford the title product as a colourless liquid (43g, 96.5%)

### Description 2: (±){1-[Amino(phenyl)methyl]cyclopentyl}dimethylamine

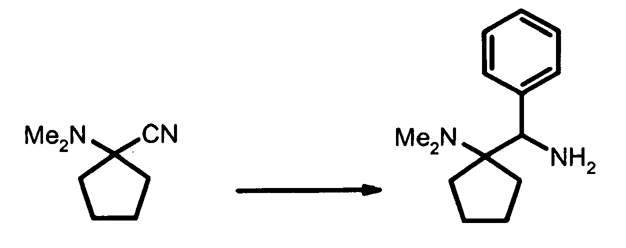

To a solution of 1-(dimethylamino)cyclopentanecarbonitrile D1 (4g; 28.9mmol) in THF (30ml) at -70°C under argon was added dropwise a solution of phenyllithium in dibutylether (17.7ml of a 1.8M solution; 32mmol). The reaction mixture was allowed to warm to room temperature over 3h., recooled to 0°C and methanol added (30ml) followed by careful addition of sodium borohydride (3.3g; 87mmol). After stirring at room temperature for 18h, the reaction mixture was cooled to 0°C and saturated aqueous sodium bicarbonate added. The organic phase was evaporated and the resulting slurry extracted three times with DCM (3 x 150ml). The combined extracts were dried (Na₂SO₄), and evaporated to afford the crude product as a yellow oil (6.8g). Half of the crude product was chromatographed on silica gel eluting with DCM-methanol (9/1) and DCM-2M ammonia in methanol mixtures (95/5 to 9/1 to 8/2) to afford the title product as a colourless oil (2.3g). This oil was dissolved in diethyl ether (20ml) and hydrogen chloride in diethylether was added at 0°C. After 20min, the suspension was filtered to give the dihydrochloride salt (2.8g) of the title product as a white solid. ¹H NMR (DMSO) 8:1.25 (2H, bs), 1.36 (2H, bs), 1.66 - 2.13 (4H, m), 2.64 (3H,s), 2.79 (3H, s), 4.92 (1H, bs), 7.32 (3H, m), 7.54 (2H, m), 8.95 (3H, bs), 10.82 (1H, bs).

### Alternative method:

To a solution of 1-(dimethylamino)cyclopentanecarbonitrile D1 (43g; 311mmol) in THF (1L) at -70°C under argon was added dropwise a solution of phenyllithium in dibutylether (346ml of a 1.8M solution; 622mmol) over 10 minutes. The reaction mixture was stirred at -70°C for 2h, then allowed to warm to room temperature and it was stirred overnight. The reaction mixture was cooled in ice and saturated aqueous sodium bicarbonate solution was added. The mixture was stirred for 30 minutes, separated and the aqueous layer extracted with diethyl ether. The combined organics were dried (Na₂SO₄) and concentrated under reduced pressure to give an oil.
The oil was dissolved in methanol (1.2L) and cooled in ice. Sodium borohydride (20g) was added in 4 portions over 5 minutes and the mixture was stirred for half an hour with ice cooling. The cooling was then removed and stirring was continued at room temperature for one and a half hours. The reaction mixture was then ice cooled and water was added. The resultant mixture was evaporated *in vacuo* and fractioned between 2N HCl and ethyl acetate. The organics were extracted with 2N HCl. The combined acid extracts were washed with ethyl acetate, basified with NaOH and extracted into DCM. The combined DCM extracts were dried (Na₂SO₄), and evaporated *in vacuo* to afford the product as a pale green liquid (64.66g, 95.4%).

### Description 3: {1-[Amino(phenyl)methyl]cyclopentyl}dimethylamine Enantiomer 1 and Enantiomer 2

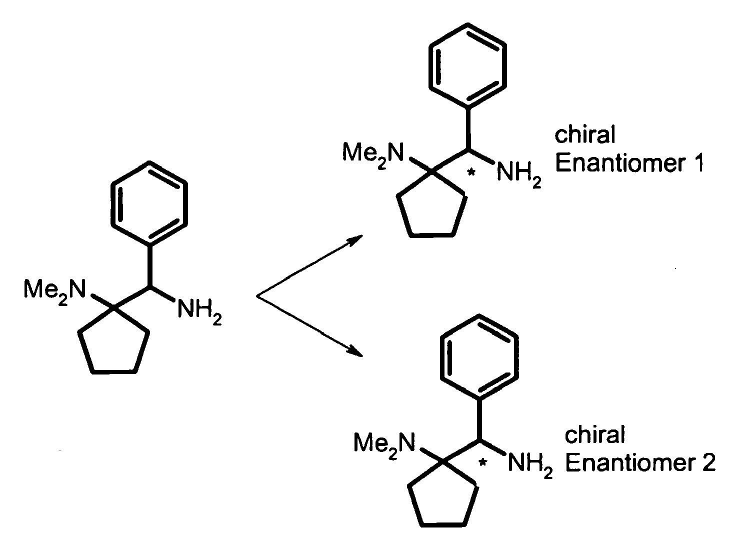

### Method 1.

Racemic {1-[amino(phenyl)methyl]cyclopentyl}dimethylamine D2 (0.6g; 2.75mmol) was separated by semi-preparative chiral HPLC using the conditions described below to afford the title products, enantiomer 1, (0.27g); Chiral HPLC: 98% ee; ¹H NMR (CDCl3) δ: 0.42 (1H, m), 1.32 (3H, m), 1.49 (1H, m), 1.63 (1H, m), 1.76 (1H, m), 1.95 (3H, m), 2.29 (6H, s), 4.39 (1H, s), 7.28 (3H, m), 7.50 (2H, d); Mass spectrum (Electrospray LC/MS): Found 219 (MH⁺). C₁₄H₂₂N₂ requires 218; and enantiomer 2 (0.28g); Chiral HPLC: 98% ee; ¹H NMR (CDCl3) δ: 0.42 (1H, m), 1.32 (3H, m), 1.49 (1H, m), 1.63 (1H, m), 1.76 (1H, m), 1.95 (3H, m), 2.29 (6H, s), 4.39 (1H, s), 7.28 (3H, m), 7.50 (2H, d); Mass spectrum (Electrospray LC/MS): Found 219 (MH⁺). C₁₄H₂₂N₂ requires 218.

Semi-preparative HPLC conditions:

| | |
|---|---|
| Column: | Chiralpak AD-H 5µm, 250 x 21 mm |
| Mobile phase: | A: n-Hexane; B: Ethanol + 0.1 %isopropylamine |
| Gradient: | isocratic 5%B |
| Flow rate: | 7ml/min |
| UV wavelength range: | 225nm |
| Elution time: | 30min |

**Analytical chromatography conditions:**

| | |
|---|---|
| Column: | chiralpak AD-H 5 um, 250 x 4.6 mm |
| Mobile phase: | A: n-Hexane; B: Ethanol + 0.1 % isopropyl amine |
| Gradient: | isocratic 5% B |
| Flow rate: | 1 ml/min |
| UV wavelength range: | 200-400 nm |
| Analysis time: | 10 min |
| Ret. Time: | 5.9min (Enantiomer 1); 7.6min (Enantiomer 2) |

### Method 2.

Salt formation:
To a solution of racemic {1-[amino(phenyl)methyl]cyclopentyl}dimethylamine D2 (16.9g; 77.5mmol) in isopropanol (170ml) at 50 °C, was added dropwise a solution of (R)-methoxy phenylacetic acid (12.84g; 77.5mmol) in isopropanol (75ml). After 20 min the mixture was cooled to room temperature and left stirring for a further 4h. The precipitated solid was collected by filtration (13.42g).

### Regeneration of chiral free base:

The solid was then treated with 1 M NaOH (50ml) and DCM (167ml). The phases were separated and the aqueous phase washed with DCM (4x167ml). The combined organic phases were washed with 1 M NaOH (2x35ml), then brine (100ml), dried (Na₂SO₄), and concentrated to yield the title compound, enantiomer 2, as a colourless oil (7.6g). Chiral HPLC: >96% ee; ¹H NMR (CDCl3) δ: 0.42 (1H, m), 1.32 (3H, m), 1.49 (1H, m), 1.63 (1H, m), 1.76 (1H, m), 1.95 (3H, m), 2.29 (6H, s), 4.39 (1H, s), 7.28 (3H, m), 7.50 (2H, d); Mass spectrum (Electrospray LC/MS): Found 219 (MH⁺). C₁₄H₂₂N₂ requires 218.

Heterocyclic carboxylic acids were obtained commercially or synthesised by known methods.

### Example 1: (±)-N-[[1-(Dimethylamino)cyclopentyl](phenyl)methyl]-5-(methyloxy)-1-benzofuran-4-carboxamide

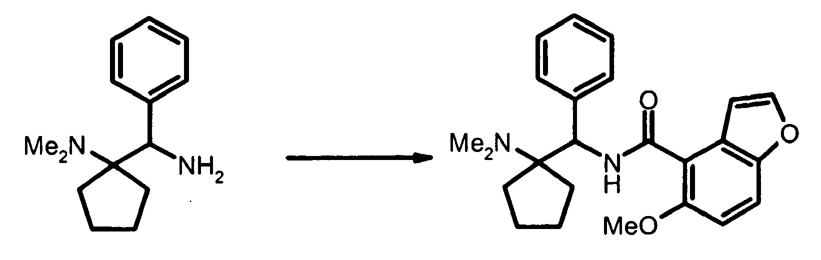

(±){1-[Amino(phenyl)methyl]cyclopentyl}dimethylamine D2 (75mg; 0.344mmol), 5-methoxy-benzofuran-4-carboxylic acid (86mg; 0.447mmol), HOBt (68mg; 0.447mmol) and PS-DCC (350mg of 1.3 mmol/g loading, 0.447mmol) in DCM (4.5ml) and DMF (0.5ml) were shaken for 66h. The mixture was filtered, spent resin washed with DCM and the filtrates and washings combined and stirred for 30min with saturated sodium bicarbonate. The layers were separated using a phase separation cartridge and the organic layer applied directly onto an SCX cartridge (2g). Washing with DCM (2 column volumes), 50% DCM/methanol (1 column volume) and methanol (2 column volumes), followed by elution with 1 M ammonia in methanol (2 column volumes) and evaporation afforded the title product. ¹H NMR (CDCl₃) δ: 1.10 - 1.20 (1H, m), 1.4 - 1.7 (4H, m), 1.75 - 1.90 (3H, m), 2.31 (6H, s), 4.07 (3H, s), 5.34 (1H, d, J = 7Hz), 7.01 (1H, d, J = 8Hz), 7.19 - 7.23 (1H, m), 7.29 - 7.30 (2H, m), 7.41 - 7.43 (2H, m), 7.52 - 7.54 (1H, m), 7.61 (2H, m), 9.06 (1H, d, J = 7Hz). Mass Spectrum (Electrospray LC/MS): Found 393 (MH⁺). C₂₄H₂₈N₂O₃ requires 392; Ret. time 2.08min. This product was dissolved in 25% methanol/DCM (2ml) and 1M HCl in ether (1ml) added. Evaporation afforded the hydrochloride salt of the title product (145mg; 98%).

### Example 2: (±)-N-[[1-(Dimethylamino)cyclopentyl](phenyl)methyl]-7-(methyloxy)-1-benzofuran-4-carboxamide

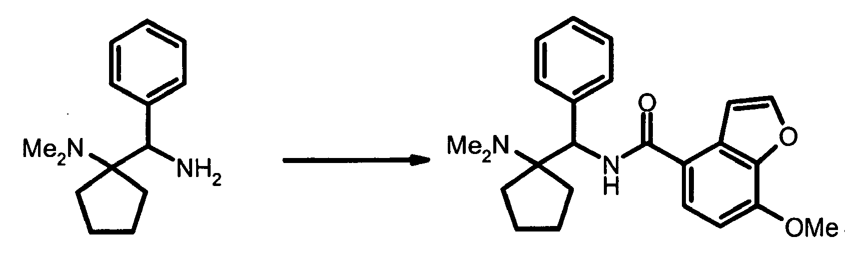

To a solution of 7-(methyloxy)-1-benzofuran-4-carboxylic acid (20.17mg; 0.105mmol) in DCM (2ml) and NMP (0.5ml) was added HOBt (18mg; 0.133mmol) and PL-DCC (88mg; 0.140mmol; Polymer Labs 1.59mmol/g). The mixture was shaken at room temperature for 90min and then 1-[amino(phenyl) methyl]cyclopentyl}dimethylamine D2 dihydrochloride (20mg; 0.070mmol) and PS-diisopropylethylamine (82mg; 0.212mmol; Polymer Labs 2.59mmol/g) were then added and shaking continued for 18h at room temperature in darkness. PS-Trisamine (85mg; ca.0.350mmol; Argonaut 4.11 mmol/g) was then added and after shaking for a further 3h, the mixture was filtered and the resins washed well with DCM and methanol. The filtrate was reduced in volume by evaporation *in vacuo* and loaded onto an SCX cartridge (500mg). Washing with DCM, then methanol followed by elution with 1 M ammonia in methanol afforded the title product (27.9mg).

**Analytical chromatography conditions:**

| | |
|---|---|
| Column: | X Terra MS C18 5 um, 50 x 4.6 mm |
| Mobile phase: | A: NH₄HCO₃ sol. 10 mM, pH10; B: acetonitrile |
| Gradient: | 30% (B) for 1 min, from 30% (B) to 95% (B) in 9 min, 95% |
| | (B) for 3 min Flow rate: 1 ml/min |
| UV wavelength range: | 210-350 nm |
| Mass range: | 100-900 amu |
| Ionization: | ES+; Found 393 (MH⁺). C₂₄H₂₈N₂O₃ requires 392. |
| | Ret. time 7.57 min Purity: 100%. |
| Mass Spectrum (Electrospray LC/MS): Found 393 (MH⁺). C₂₄H₂₈N₂O₃ requires 392; Ret. time 1.92min. | |

### Example 3: N-[[1-(Dimethylamino)cyclopentyl](phenyl)methyl]-2,2-dimethyl-1,3-benzodioxole-5-carboxamide chiral

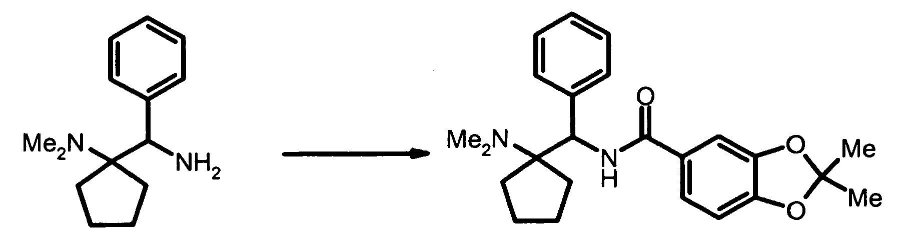

To PS-EDC (0.068g; 0.10mmol; 1.42mmol/g) was added a solution of HOAt (0.01 mmol in 0.8ml (THF:DCM,1:1)) followed by the addition of 2,2-dimethyl-1,3-benzodioxole-5-carboxylic acid (0.010g; 0.05mmol) in 1:3 NMP:THF(0.25ml) and then {1-[amino(phenyl)methyl]cyclopentyl}dimethylamine D3 enantiomer 2 (0.011g 0.05mmol) in DCM (0.25ml). The reaction was allowed to mix for 60h. Following this PS-isocyanate (0.068g, 0.1mmol, 1.5mmol/g) and PS-C03 (0.068g, 0.1mmol, 1.5mmol/g) were added and allowed to mix for another 24h. The reaction mixture was filtered and passed through an SCX block (500mg) (pre- wetted with DCM). The content of the Robbins block was washed with more solvent (DCM:THF, 1:1) and allowed to pass through the SCX which was then washed with DCM (2ml x2) and methanol (2ml x 2). The SCX was then eluted with 0.5M ammonia in methanol and the product containing eluent evaporated to afford the title product (20.7mg; 100%). Mass Spectrum (Electrospray LC/MS): Found 395 (MH⁺). C₂₄H₃₀N₂O₃ requires 394; Ret. time 2.03min.

### Example 4: N-[[1-(Dimethylamino)cyclopentyl](phenyl)methyl]-1-benzofuran-3-carboxamide chiral

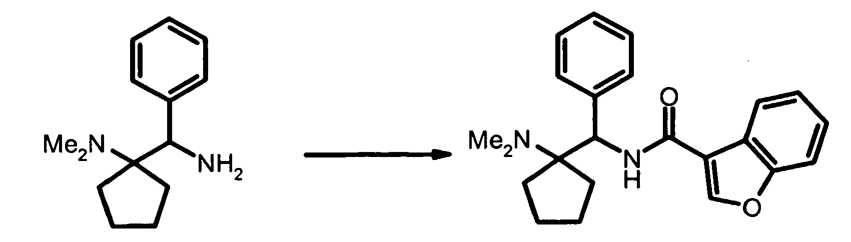

The title compound (20.5mg; 100%) was prepared from {1-[amino(phenyl)methyl] cyclopentyl}dimethylamine D3 enantiomer 2 (0.011g 0.05mmol) and benzofuran-3-carboxylic acid (0.008g; 0.05mmol) using the method described in E3. Mass Spectrum (Electrospray LC/MS): Found 363 (MH⁺). C₂₃H₂₆N₂O₂ requires 362; Ret. time 2.02min.

The compounds in the Table below were prepared using similar methods to those described for the Examples above. Coupling method: P = Polymer-supported DCC (using method similar to that in Example 2). Work-up and purification was carried out using appropriate methods similar to those described in the examples above.

| **Ex** | **Structure** | **Method** | **Mass spectrum (Electrospray LC/MS), API⁺ Ret.time (min)** | **Name** |
|---|---|---|---|---|
| 5 | 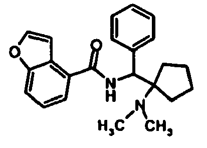 | P | Found 363(MH⁺) C₂₃H₂₆N₂O₂ requires 362; 1.98 | ± *N*-[[1-(dimethylamino) cyclopentyl](phenyl)methyl]-1-benzofuran-4-carboxamide |

The compounds of the Examples above may be converted to their corresponding hydrochloride salts by dissolving the parent free base in DCM or DCM / methanol mixtures and adding 1 M hydrogen chloride in ether, followed by evaporation and drying *in vacuo.*

## Claims

1. A compound of formula (I) or a salt or solvate thereof: 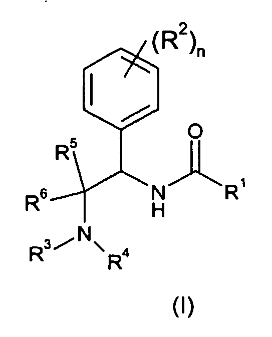 wherein:
R¹ is a group selected from: 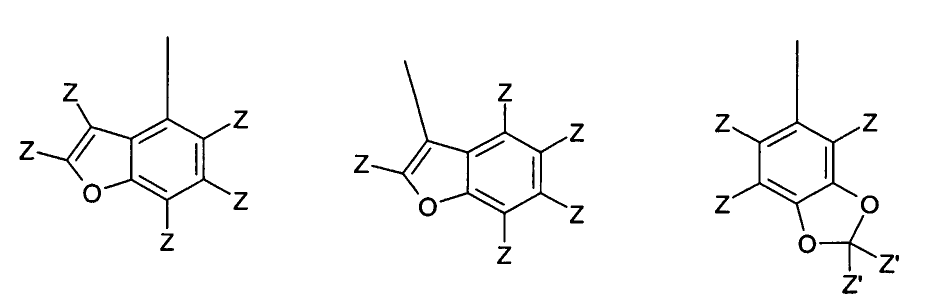 and wherein
Each Z is independently selected from hydrogen, halogen, C₃₋₇cycloalkyl, C₁₋₄alkyl, haloC₁₋₄alkyl, C₁₋₄alkoxyC₁₋₄alkyl, C₁₋₄alkoxy, haloC₁₋₄alkoxy, C₁₋₄alkylthio, haloC₁₋₄alkylthio, C₃₋₇cycloalkylC₁₋₄alkoxy, C₁₋₄alkylsulphoxy, C₁₋₄ alkylsulphonyl, and cyano;
and each Z' is independently selected from hydrogen, fluoro or C₁₋₄alkyl;
R² is selected from the group consisting of halogen, cyano, C₁₋₄alkoxy, C₁₋₄alkyl, haloC₁₋₄alkyl, haloC₁₋₄alkoxy, C₃₋₇cycloalkyl, C(O)NR⁹R¹⁰, (where each of R⁹ and R¹⁰ is independently hydrogen or C₁₋₄alkyl, or R⁹ and R¹⁰ together with the nitrogen atom to which they are attached form a 4-, 5-, 6- or 7-membered saturated carbocyclic ring, the 4-, 5-, 6- or 7-membered saturated ring optionally further comprising an additional heteroatom group selected from O, N and S(O)ₘ (where m is 0, 1, or 2)), C₃₋₇cycloalkylC₁₋₄alkoxy, C₁₋₄alkylthio, and haloC₁₋₄alkylthio;
n is 0, 1 or 2.
R³ and R⁴ are independently selected from hydrogen, C₁₋₄alkyl, optionally substituted with one or more groups Y; or R³ and R⁴ together with the nitrogen atom to which they are attached form a saturated or partially unsaturated carbocyclic 4-, 5- 6-or 7-membered ring optionally substituted with a group Y';
Y is selected from the group consisting of C₁₋₄alkoxy, hydroxy, haloC₁₋₄alkoxy and C₃₋₅cycloalkyl and C₅₋₁₀ aryl;
Y' is selected from the group consisting of C₁₋₄alkyl, C₁₋₄alkoxy, halogen, hydroxy, haloC₁₋₄alkoxy, C₃₋₅cycloalkyl and C₅₋₁₀aryl or Y' forms a -CH₂- or -CH₂-CH₂- bridge between two atoms on the 4-, 5- or 6- membered ring;
R⁵ and R⁶ are independently C₁₋₄alkyl, optionally substituted with one or more groups X; or R⁵ and R⁶ together with the carbon atom to which they are attached form a saturated 5- or 6-membered carbocyclic ring optionally substituted with one or more groups X', in the case of R⁵ and R⁶ together with the carbon atom to which they are attached forming a 5-membered saturated carbocyclic ring, that ring may optionally further comprise an additional heteroatom group selected from O, N and S(O)ₘ; where m = 0, 1 or 2.
X is selected from the group consisting of halogen, hydroxy, C₁₋₄alkoxy, haloC₁₋₄alkyl, haloC₁₋₄alkoxy and C₅₋₁₀aryl; and
X' is selected from the group consisting of halogen, hydroxy, C₁₋₄alkoxy, haloC₁₋₄alkyl, haloC₁₋₄alkoxy and C₅₋₁₀aryl.

2. A compound as claimed in claim 1 which is a compound of formula (1a) or a salt or solvate thereof: 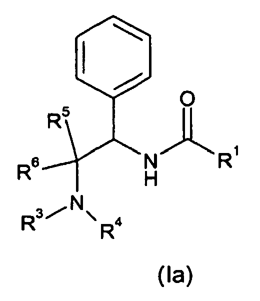 wherein:
R¹ is a group selected from: 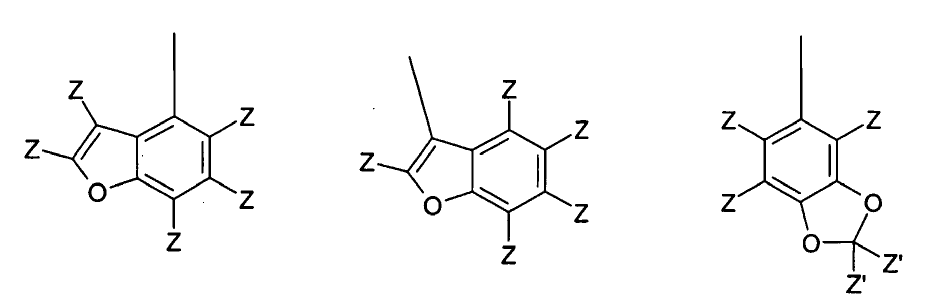 wherein
each Z is independently selected from the group consisting of hydrogen, halogen, cyano, C₁₋₄alkyl, C₁₋₄alkoxy, haloC₁₋₄alkyl, haloC₁₋₄alkoxy, C₁₋₄alkoxyC₁₋₄alkyl, C₁₋₄alkylthio, C₃₋₆cycloalkyl, ;
each Z' is methyl;
R³ and R⁴ are independently selected from hydrogen, C₁₋₄alkyl (for example methyl and ethyl), optionally substituted with a group Y, or R³ and R⁴ together with the nitrogen atom to which they are attached form a saturated or partially unsaturated (for example saturated) 4-, 5-, 6- or 7-membered carbocyclic ring optionally substituted with a group Y';
Y is selected from the group consisting of C₁₋₄alkoxy, hydroxyl, C₃₋₅cycloalkyl and C₅₋₁₀aryl;
Y' is selected from the group consisting of halogen and C₁₋₄alkyl;
R⁵ and R⁶ are independently selected from methyl and ethyl, optionally substituted with one or more groups X; or R⁵ and R⁶ together with the carbon atom to which they are attached form a saturated 5- or 6-membered carbocyclic ring and in the case of R⁵ and R⁶ together with the carbon atom to which they are attached forming a carbocyclic 5-membered saturated ring, that ring may optionally further comprise an oxygen heteroatom; and
X is selected from the group consisting of hydroxy and C₁₋₄alkoxy.

3. A compound as claimed in claim 1 or claim 2 which is any of Examples 1 to 5 or a salt or solvate thereof.

4. A method of preparing a compound as defined in any one of claims 1 to 3, comprising the step of reacting a compound of formula (II): 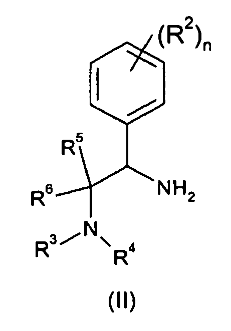 wherein n, R², R³, R⁴, R⁵ and R⁶ are as defined in formula (I) in any one of claims 1 to 3, with a compound of formula (III): 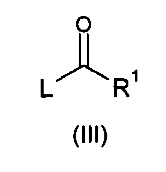 wherein R¹ is as defined in formula (I) as claimed in any one of claims 1 to 3 and L represents a suitable leaving group;
and thereafter optionally:
• removing any protecting groups and/or
• converting a compound of formula (I) into another compound of formula (I) and/or
• forming a salt or solvate.

5. A compound as claimed in any of claims 1-3 for use in therapy.

6. A compound as claimed in any of claims 1-3 for use in the treatment of a disorder mediated by GlyT1.

7. A compound as claimed in claim 6, wherein the disorder is psychosis, including schizophrenia, dementia or attention deficit disorder.

8. A compound according to claims 1-3 for one in the treatment of a mammal, including a human, suffering from or susceptible to a disorder mediated by GlyT1, which comprises administering an effective amount of a compound as claimed in any of claims 1-3.

9. A compound as claimed in claim 8, wherein the disorder is psychosis, including schizophrenia, dementia or attention deficit disorder.

10. Use of a compound as claimed in any of claims 1-3 in the preparation of a medicament for the treatment of a disorder mediated by GlyT1.

11. Use as claimed in claim 10, wherein the disorder is psychosis, including schizophrenia, dementia or attention deficit disorder.

12. A pharmaceutical composition comprising a compound as claimed in any of claims 1-3, and at least one pharmaceutically acceptable carrier, diluent or excipient.

13. A pharmaceutical composition as claimed in claim 12 further comprising one or more other therapeutic agents, selected from antidepressant agents selected from 5HT3 antagonists, serotonin agonists, NK-1 antagonists, selective serotonin reuptake inhibitors (SSRI), noradrenaline re-uptake inhibitors (SNRI), tricyclic antidepressants, dopaminergic antidepressants, H3 antagonists, 5HT1A antagonists, 5HT1B antagonists, 5HT1D antagonists, D1 agonists, M1 agonists; anticonvulsant agents; atypical antipsychotic drugs and cognitive enhancers.

## Patentansprüche

1. Eine Verbindung der Formel (I) oder ein Salz oder Solvat davon: 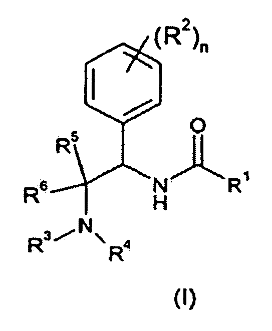 wobei:
R¹ ein Rest, ausgewählt aus: 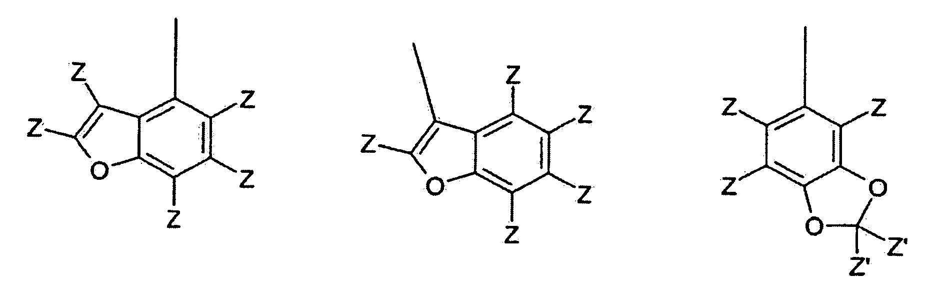 ist, und wobei
jedes Z unabhängig aus Wasserstoff, Halogen, C₃₋₇-Cycloalkyl, C₁₋₄-Alkyl, Halogen-C₁₋₄-alkyl, C₁₋₄-Alkoxy-C₁₋₄-alkyl, C₁₋₄-Alkoxy, Halogen-C₁₋₄-alkoxy, C₁₋₄-Alkylthio, Halogen-C₁₋₄-alkylthio, C₃₋₇-Cycloalkyl-C₁₋₄-alkoxy, C₁₋₄-Alkylsulfoxy, C₁₋₄-Alkylsulfonyl und Cyano ausgewählt ist;
und jedes Z' unabhängig aus Wasserstoff, Fluor oder C₁₋₄-Alkyl ausgewählt ist;
R² aus der Gruppe, bestehend aus Halogen, Cyano, C₁₋₄-Alkoxy, C₁₋₄-Alkyl, Halogen-C₁₋₄-alkyl, Halogen-C₁₋₄-alkoxy, C₃₋₇-Cycloalkyl, C(O)NR⁹R¹⁰ (wobei jedes von R⁹ und
R¹⁰ unabhängig Wasserstoff oder C₁₋₄-Alkyl ist oder R⁹ und R¹⁰ zusammen mit dem Stickstoffatom, an das sie gebunden sind, einen 4-, 5-, 6- oder 7-gliedrigen gesättigten carbocyclischen Ring bilden, wobei der 4-, 5-, 6- oder 7-gliedrige gesättigte Ring gegebenenfalls weiter einen zusätzlichen Heteroatomrest, ausgewählt aus O, N und S(O)ₘ (wobei m 0, 1 oder 2 ist) umfasst), C₃₋₇-Cycloalkyl-C₁₋₄-alkoxy, C₁₋₄-Alkylthio und Halogen-C₁₋₄-alkylthio, ausgewählt ist;
n 0, 1 oder 2 ist;
R³ und R⁴ unabhängig aus Wasserstoff, C₁₋₄-Alkyl, gegebenenfalls substituiert mit einem oder mehreren Resten Y, ausgewählt sind; oder R³ und R⁴ zusammen mit dem Stickstoffatom, an das sie gebunden sind, einen gesättigten oder teilweise ungesättigten, carbocyclischen, 4-, 5-, 6- oder 7-gliedrigen Ring bilden, der gegebenenfalls mit einem Rest Y' substituiert ist;
Y aus der Gruppe, bestehend aus C₁₋₄-Alkoxy, Hydroxy, Halogen-C₁₋₄-alkoxy und C₃₋₅-Cycloalkyl und C₅₋₁₀-Aryl, ausgewählt ist;
Y' aus der Gruppe, bestehend aus C₁₋₄-Alkyl, C₁₋₄-Alkoxy, Halogen, Hydroxy, Halogen-C₁₋₄-alkoxy, C₃₋₅-Cycloalkyl und C₅₋₁₀-Aryl, ausgewählt ist oder Y' eine -CH₂- oder -CH₂-CH₂- Brücke zwischen zwei Atomen an dem 4-, 5- oder 6-gliedrigen Ring bildet;
R⁵ und R⁶ unabhängig C₁₋₄-Alkyl, gegebenenfalls substituiert mit einem oder mehreren Resten X, sind; oder R⁵ und R⁶ zusammen mit dem Kohlenstoffatom, an das sie gebunden sind, einen gesättigten 5- oder 6-gliedrigen carbocyclischen Ring bilden, der gegebenenfalls mit einem oder mehreren Resten X' substituiert ist, falls R⁵ und R⁶
zusammen mit dem Kohlenstoffatom, an das sie gebunden sind, einen 5-gliedrigen gesättigten carbocyclischen Ring bilden, dieser Ring gegebenenfalls weiter einen zusätzlichen Heteroatomrest, ausgewählt aus O, N und S(O)ₘ, wobei m = 0, 1 oder 2, umfassen kann;
X aus der Gruppe, bestehend aus Halogen, Hydroxy, C₁₋₄-Alkoxy, Halogen-C₁₋₄-alkyl, Halogen-C₁₋₄-alkoxy und C₅₋₁₀-Aryl, ausgewählt ist; und
X' aus der Gruppe, bestehend aus Halogen, Hydroxy, C₁₋₄-Alkoxy, Halogen-C₁₋₄-alkyl, Halogen-C₁₋₄-alkoxy und C₅₋₁₀-Aryl, ausgewählt ist.

2. Eine Verbindung wie in Anspruch 1 beansprucht, wobei es sich um eine Verbindung der Formel (Ia) oder ein Salz oder Solvat davon handelt: 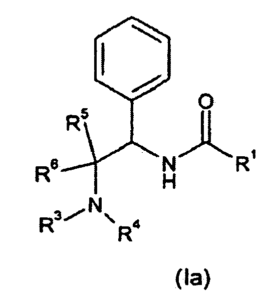 wobei
R¹ ein Rest, ausgewählt aus: 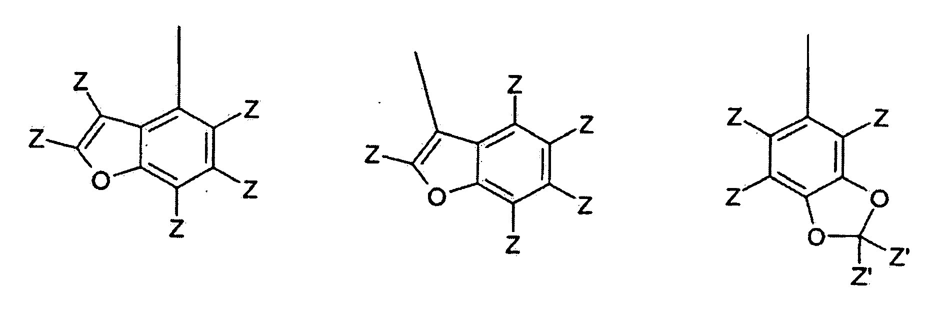 ist, wobei
jedes Z unabhängig aus der Gruppe, bestehend aus Wasserstoff, Halogen, Cyano, C₁₋₄-Alkyl, C₁₋₄-Alkoxy, Halogen-C₁₋₄-alkyl, Halogen-C₁₋₄-alkoxy, C₁₋₄-Alkoxy-C₁₋₄-alkyl, C₁₋₄-Alkylthio, C₃₋₆-Cycloalkyl, ausgewählt ist;
jedes Z' Methyl ist;
R³ und R⁴ unabhängig aus Wasserstoff, C₁₋₄-Alkyl (z.B. Methyl und Ethyl), gegebenenfalls substituiert mit einem Rest Y, ausgewählt sind, oder R³ und R⁴ zusammen mit dem Stickstoffatom, an das sie gebunden sind, einen gesättigten oder teilweise ungesättigten (z.B. gesättigten) 4-, 5-, 6- oder 7-gliedrigen carbocyclischen Ring bilden, der gegebenenfalls mit einem Rest Y' substituiert ist;
Y aus der Gruppe, bestehend aus C₁₋₄-Alkoxy, Hydroxyl, C₃₋₅-Cycloalkyl und C₅₋₁₀-Aryl, ausgewählt ist;
Y' aus der Gruppe, bestehend aus Halogen und C₁₋₄-Alkyl, ausgewählt ist;
R⁵ und R⁶ unabhängig aus Methyl und Ethyl, gegebenenfalls substituiert mit einem oder mehreren Resten X, ausgewählt sind; oder R⁵ und R⁶ zusammen mit dem Kohlenstoffatom, an das sie gebunden sind, einen gesättigten 5- oder 6-gliedrigen carbocyclischen Ring bilden, und falls R⁵ und R⁶ zusammen mit dem Kohlenstoffatom, an das sie gebunden sind, einen carbocyclischen 5-gliedrigen gesättigten Ring bilden, dieser Ring gegebenenfalls weiter ein Sauerstoff-Heteroatom umfassen kann; und
X aus der Gruppe, bestehend aus Hydroxy und C₁₋₄-Alkoxy, ausgewählt ist.

3. Eine Verbindung wie in Anspruch 1 oder Anspruch 2 beansprucht, bei welcher es sich um eines der Beispiele 1 bis 5 oder ein Salz oder Solvat davon handelt.

4. Ein Verfahren zur Herstellung einer Verbindung wie in einem der Ansprüche 1 bis 3 definiert, umfassend den Schritt des Umsetzens einer Verbindung der Formel (II): 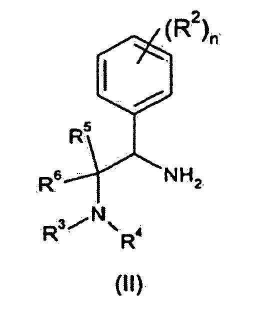 wobei n, R², R³, R⁴, R⁵ und R⁶ wie in Formel (I) in einem der Ansprüche 1 bis 3 definiert sind, mit einer Verbindung der Formel (III): 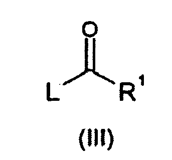 wobei R¹ wie in Formel (I) wie in einem der Ansprüche 1 bis 3 beansprucht definiert ist und L eine geeignete Abgangsgruppe darstellt;
und danach gegebenenfalls:
• Entfernen jeglicher Schutzgruppen und/oder
• Umwandeln einer Verbindung der Formel (I) in eine andere Verbindung der Formel
(I) und/oder
• Bilden eines Salzes oder Solvats.

5. Eine Verbindung wie in einem der Ansprüche 1 bis 3 beansprucht zur Verwendung in der Therapie.

6. Eine Verbindung wie in einem der Ansprüche 1 bis 3 beansprucht zur Verwendung bei der Behandlung einer durch GlyT1 vermittelten Störung.

7. Eine Verbindung wie in Anspruch 6 beansprucht, wobei die Störung Psychose, einschließlich Schizophrenie, Demenz oder Aufmerksamkeitsdefizitsyndrom ist.

8. Eine Verbindung nach den Ansprüchen 1 bis 3 zur Verwendung bei der Behandlung eines Säugers, einschließlich eines Menschen, der an einer durch GlyT1 vermittelten Störung leidet oder dafür empfänglich ist, umfassend das Verabreichen einer wirksamen Menge einer Verbindung wie in einem der Ansprüche 1 bis 3 beansprucht.

9. Eine Verbindung wie in Anspruch 8 beansprucht, wobei die Störung Psychose, einschließlich Schizophrenie, Demenz oder Aufmerksamkeitsdefizitsyndrom ist.

10. Verwendung einer Verbindung wie in einem der Ansprüche 1 bis 3 beansprucht bei der Herstellung eines Medikaments zur Behandlung einer durch GlyT1 vermittelten Störung.

11. Verwendung wie in Anspruch 10 beansprucht, wobei die Störung Psychose, einschließlich Schizophrenie, Demenz oder Aufmerksamkeitsdefizitsyndrom ist.

12. Ein Arzneimittel, umfassend eine Verbindung wie in einem der Ansprüche 1 bis 3 beansprucht und mindestens einen/ein pharmazeutisch verträglichen/verträgliches Träger, Verdünnungsmittel oder Exzipienten.

13. Ein Arzneimittel wie in Anspruch 12 beansprucht, weiter umfassend ein oder mehrere andere therapeutische Mittel, ausgewählt aus Antidepressiva, ausgewählt aus 5HT3-Antagonisten, Serotonin-Agonisten, NK-1-Antagonisten, selektiven Serotoninwiederaufnahmehemmern (SSRI), Noradrenalinwiederaufnahmehemmern (SNRI), tricyclischen Antidepressiva, dopaminergenen Antidepressiva, H3-Antagonisten, 5HT1A-Antagonisten, 5HT1B-Antagonisten, 5HT1D-Antagonisten, D1-Agonisten, M1-Agonisten; krampflösenden Mitteln; atypischen antipsychotischen Arzneistoffen und die Wahrnehmung verstärkenden Mitteln.

## Revendications

1. Composé de formule (I) ou un de ses sels ou produits de solvatation : 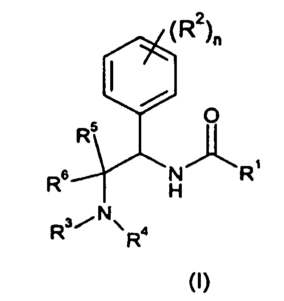 formule dans laquelle :
R¹ représente un groupe choisi entre : 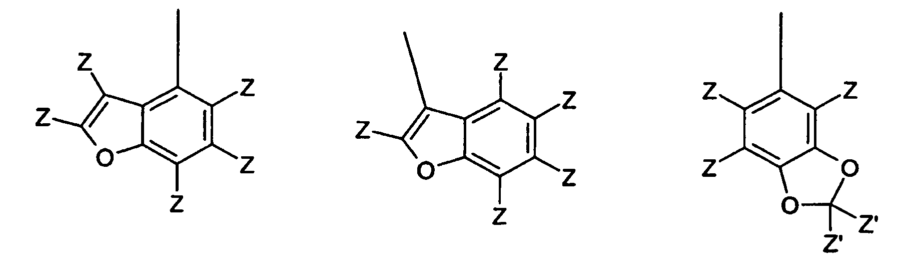 et dans lequel
chaque Z est choisi indépendamment entre un atome d'hydrogène, des groupes halogéno, cycloalkyle en C₃ à C₇, alkyle en C₁ à C₄, halogénalkyle en C₁ à C₄, (alkoxy en C₁ à C₄) (alkyle en C₁ à C₄), alkoxy en C₁ à C₄, halogénalkoxy en C₁ à C₄, alkylthio en C₁ à C₄, halogénalkylthio en C₁ à C₄, (cycloalkyle en C₃ à C₇) (alkoxy en C₁ à C₄), alkylsulfoxy en C₁ à C₄, alkylsulfonyle en C₁ à C₄ et cyano ;
et chaque Z' est choisi indépendamment entre un atome d'hydrogène, un groupe fluoro et un groupe alkyle en C₁ à C₄ ;
R² est choisi dans le groupe consistant en des groupes halogéno, cyano, alkoxy en C₁ à C₄, alkyle en C₁ à C₄, halogénalkyle en C₁ à C₄, halogénalkoxy en C₁ à C₄, cycloalkyle en C₃ à C₇, C(O)NR⁹R¹⁰, (dans lequel chacun de R⁹ et R¹⁰ représente indépendamment un atome d'hydrogène ou un groupe alkyle en C₁ à C₄, ou bien R⁹ et R¹⁰, conjointement avec l'atome d'azote auquel ils sont fixés, forment un noyau carbocyclique tétra-, penta-, hexa- ou heptagonal saturé, le noyau tétra-, penta-, hexa- ou heptagonal saturé comprenant en outre un groupe hétéroatomique supplémentaire choisi entre O, N et S(O)ₘ (dans lequel m est égal à 0, 1 ou 2)), (cycloalkyle en C₃ à C₇)(alkoxy en C₁ à C₄), alkylthio en C₁ à C₄ et halogénalkylthio en C₁ à C₄ ;
n est égal à 0, 1 ou 2 ;
R³ et R⁴ sont choisis indépendamment entre un atome d'hydrogène et un groupe alkyle en C₁ à C₄ facultativement substitué avec un ou plusieurs groupes Y ; ou bien R³ et R⁴, conjointement avec l'atome d'azote auquel ils sont fixés, forment un noyau carbocyclique tétra-, penta-, hexa- ou heptagonal saturé ou partiellement insaturé facultativement substitué avec un groupe Y' ;
Y est choisi dans le groupe consistant en des groupes alkoxy en C₁ à C₄, hydroxy, halogénalkoxy en C₁ à C₄, cycloalkyle en C₃ à C₅ et aryle en C₅ à C₁₀ ;
Y' est choisi dans le groupe consistant en des groupes alkyle en C₁ à C₄, alkoxy en C₁ à C₄, halogéno, hydroxy, halogénalkoxy en C₁ à C₄, cycloalkyle en C₃ à C₅ et aryle en C₅ à C₁₀ ou bien Y' forme un pont -CH₂- ou -CH₂-CH₂- entre des deux atomes sur le noyau tétra- penta- ou hexagonal ;
R⁵ et R⁶ représentent indépendamment un groupe alkyle en C₁ à C₄, facultativement substitué avec un ou plusieurs groupes X ; ou bien R⁵ et R⁶, conjointement avec l'atome de carbone auquel ils sont fixés, forment un noyau carbocyclique penta- ou hexagonal saturé facultativement substitué avec un ou plusieurs groupes X' ; dans le cas où R⁵ et R⁶, conjointement avec l'atome de carbone auquel ils sont fixés, forment un noyau carbocyclique pentagonal saturé, ce noyau peut comprendre en outre facultativement un groupe hétéroatomique supplémentaire choisi entre O, N et S(O)m, dans lequel m est égal à 0, 1 ou 2 ;
X est choisi dans le groupe consistant en des groupes halogéno, hydroxy, alkoxy en C₁ à C₄, halogénalkyle en C₁ à C₄, halogénalkoxy en C₁ à C₄ et aryle en C₅ à C₁₀ ; et
X' est choisi dans le groupe consistant en des groupes halogéno, hydroxy, alkoxy en C₁ à C₉, halogénalkyle en C₁ à C₄, halogénalkoxy en C₁ à C₉ et aryle en C₅ à C₁₀.

2. Composé suivant la revendication 1, qui est un composé de formule (IA) ou un de ses sels ou produits de solvatation : 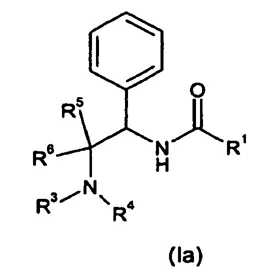 formule dans laquelle :
R¹ représente un groupe choisi entre : 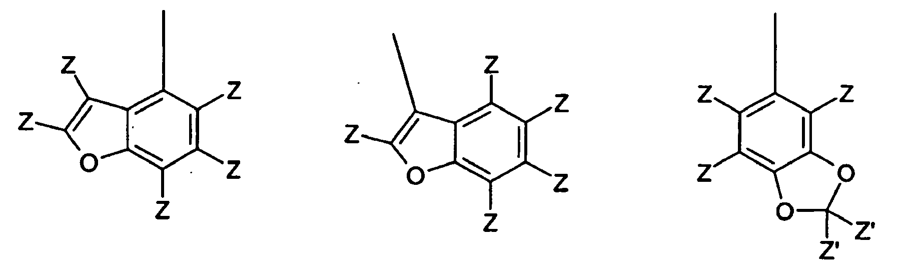 dans lequel
chaque Z est choisi indépendamment dans le groupe consistant en un atome d'hydrogène, des groupes halogéno, cyano, alkyle en C₁ à C₉, alkoxy en C₁ à C₄, halogénalkyle en C₁ à C₉, (alkoxy en C₁ à C₄) (alkyle en C₁ à C₄), alkylthio en C₁ à C₉ et cycloalkyle en C₃ à C₆ ;
chaque Z' représente un groupe méthyle ;
R³ et R⁴ sont choisis indépendamment entre un atome d'hydrogène et un groupe alkyle en C₁ à C₄ (par exemple méthyle ou éthyle) facultativement substitué avec un groupe Y ; ou bien R³ et R⁴, conjointement avec l'atome d'azote auquel ils sont fixés, forment un noyau carbocyclique tétra-, penta-, hexa- ou heptagonal saturé ou partiellement insaturé (par exemple saturé) facultativement substitué avec un groupe Y' ;
Y est choisi dans le groupe consistant en des groupes alkoxy en C₁ à C₄, hydroxyle, cycloalkyle en C₃ à C₅ et aryle en C₅ à C₁₀ ;
Y' est choisi dans le groupe consistant en des groupes halogéno et alkyle en C₁ à C₄ ;
R⁵ et R⁶ sont choisis indépendamment entre des groupes méthyle et éthyle, facultativement substitués avec un ou plusieurs groupes X, ou bien R⁵ et R⁶, conjointement avec l'atome de carbone auquel ils sont fixés, forment un noyau carbocyclique penta- ou hexagonal saturé et, dans le cas où R⁵ et R⁶, conjointement avec l'atome de carbone auquel ils sont fixés, forment un noyau carbocyclique pentagonal saturé, ce noyau peut comprendre en outre facultativement un hétéroatome d'oxygène ; et
X est choisi dans le groupe consistant en des groupes hydroxy et alkoxy en C₁ à C₄.

3. Composé suivant la revendication 1 ou la revendication 2, qui est n'importe lequel des exemples 1 à 5, ou un de ses sels ou produits de solvatation.

4. Procédé pour préparation d'un composé tel que défini dans l'une quelconque des revendications 1 à 3, comprenant l'étape de réaction d'un composé de formule (II) : 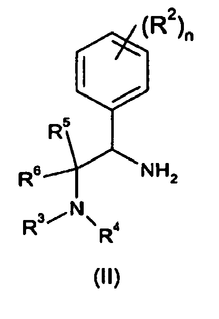 dans laquelle n, R², R³, R⁴, R⁵ et R⁶ sont tels que définis pour la formule (I) dans l'une quelconque des revendications 1 à 3, avec un composé de formule (III) : 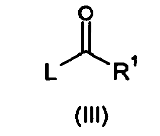 dans laquelle R¹ est tel que défini pour la formule (I) dans l'une quelconque des revendications 1 à 3 et L représente un groupe partant convenable ;
et ensuite, facultativement :
• l'élimination de n'importe quels groupes protecteurs et/ou
• la conversion d'un composé de formule (I) en un autre composé de formule (I) et/ou
• la formation d'un sel ou produit de solvatation.

5. Composé suivant l'une quelconque des revendications 1 à 3, destiné à être utilisé en thérapie.

6. Composé suivant l'une quelconque des revendications 1 à 3, destiné à être utilisé dans le traitement d'un trouble à médiation par GlyT1.

7. Composé suivant la revendication 6, dans lequel le trouble est une psychose, comprenant la schizophrénie, la démence ou le trouble de déficit d'attention.

8. Composé suivant l'une quelconque des revendications 1 à 3, destiné à être utilisé dans le traitement d'un mammifère, y compris d'un être humain souffrant d'un, ou sensible à un, trouble à médiation par GlyT1, qui comprend l'administration d'une quantité efficace d'un composé suivant l'une quelconque des revendications 1 à 3.

9. Composé suivant la revendication 8, dans lequel le trouble est une psychose, comprenant la schizophrénie, la démence ou le trouble de déficit d'attention.

10. Utilisation d'un composé suivant l'une quelconque des revendications 1 à 3, dans la préparation d'un médicament destiné au traitement d'un trouble à médiation par GlyT1.

11. Utilisation suivant la revendication 10, dans laquelle le trouble est une psychose, comprenant la schizophrénie, la démence ou le trouble de déficit d'attention.

12. Composition pharmaceutique comprenant un composé suivant l'une quelconque des revendications 1 à 3 et au moins un support, diluant ou excipient pharmaceutiquement acceptable.

13. Composition pharmaceutique suivant la revendication 12, comprenant en outre un ou plusieurs autres agents thérapeutiques, choisis entre des agents antidépresseurs choisis entre des antagonistes de 5HT3, des agonistes de sérotonine, des antagonistes de NK-1, des inhibiteurs de réabsorption de sérotonine sélectifs (SSRI), des inhibiteurs de réabsorption de noradrénaline (SNRI), des antidépresseurs tricycliques, des antidépresseurs dopaminergiques, des antagonistes de H3, des antagonistes de 5HT1A, des antagonistes de 5HT1B, des antagonistes de 5HT1D, des agonistes de D1, des agonistes de M1 ; des agents anticonvulsivants ; des médicaments antipsychotiques atypiques et des agents améliorant la cognition.
